# EUROPEAN PATENT APPLICATION

(11) **EP 3 756 720 A2**
(43) Date of publication of application: **30.12.2020**
(21) Application number: 20181583.4
(22) Date of filing: 23.06.2020
(51) Int. Cl.: A61M 25/00, A61F 2/82, A61F 2/04, A61M 39/02

(54) **IMPLANTABLE DEVICE FOR DELIVERING FLUID TO INTERNAL TARGET**

(30) Priority: 28.06.2019 US 201962868471 P; 11.06.2020 US 202016898687
(71) Applicant: Gyrus ACMI, Inc. d/b/a Olympus Surgical Technologies America, Southborough MA 01772 (US)
(72) Inventor: HOLMAN, Thomas, Princeton, Minnesota 55371 (US); MURDESHWAR, Nikhil M., Maple Grove, Minnesota 55311 (US)
(74) Representative: Noack, Andreas

(57) **Abstract**

An at least partially implantable medical device can include a body where at least a first portion of the body configured to be implanted through an ostial opening, and at least a second portion of the body configured to at least temporarily retain the body in the implanted position. The second portion can be more distal than the first portion and including a cross-sectional area larger than a cross-sectional area of the first portion. At least one of the first portion or the second portion can be configured to elute a fluid.

## Description

### CLAIM OF PRIORITY

This patent application claims the benefit of priority to U.S. Provisional Patent Application No. 62/868,471, filed June 28, 2019, which is hereby incorporated herein by reference in its entirety.

### BACKGROUND

An ostia is a small orifice or opening. A sinus cavity, pelvic opening, an ear Eustachian tube, and portions of a gastrointestinal (GI) tract, are examples of ostia. Ostia can be problematic if inflamed, partially blocked, or the like.

Chronic rhinosinusitis (CRS) is inflammation of the paranasal sinuses for a period lasting more than 12 weeks. Typically, symptoms can include, among others, nasal inflammation, anterior or posterior nasal discharge, nasal obstruction or congestion, loss of smell, and facial pain, tenderness, and swelling. CRS can be caused by an infection in the paranasal sinuses, nasal polyps, a deviated nasal septum, chronic inflammation of the sinus ostium, and allergies, among other causes. Current treatments available for treating CRS tend to focus on aerating the sinus cavity and/or applying a one-time fluid directly into the sinus cavity. For example, current treatment for CRS can include nasal, oral, or injected corticosteroids, nasal irrigation with saline, antibiotics, or surgery.

Surgery can include functional endoscopic sinus surgery (FESS). FESS can include placing an endoscope in the sinus and removing affected tissue and/or bone from the sinus and/or surrounding area and/or expanding the ostium. In the case of a deviated nasal septum, surgery can include a procedure to correct a deviated nasal septum. In the case of chronic inflammation of the sinus, surgery can include balloon sinus dilation (BSD) to better open the sinus ostium. BSD can include inserting a balloon catheter into the sinus ostium and dilating the balloon to expand the sinus ostium. A drug eluting stent can also be positioned in the sinus ostium during BSD.

### SUMMARY

The present disclosure relates to a medical device positionable inside a target anatomy (e.g., sinus ostia or other portion of the sinus, vagina or other portion of the female reproductive system, or the like), such as for aeration or optional exchange of a medium. The medical device can, for example, treat a chronic condition (e.g., CRS). The medical device can be removably connectable to a delivery system. Such systems and methods can be beneficial for treating patients (e.g., recalcitrant patients) for whom BSD can lead to undesirable side effects (e.g., pain).

According to an embodiment, a medical device insertable within a target anatomical region can include a body, at least a first portion of the body being configured to be insertable within the target anatomical region, and at least a second portion of the body being configured to facilitate retention of the body in the target anatomical region. At least one of the first portion or the second portion being configured to interact with a portion surrounding the target anatomical region and/or configured to exchange a component to effect a change within the target anatomical region.

According to an embodiment, a medical device insertable within a target anatomical region includes a body, at least a first portion of the body being configured to be insertable within a target anatomical region, and at least a second portion of the body being configured to facilitate retention of the body in the target anatomical region. At least one of the first portion or the second portion being configured to receive a medium and permit exchange thereof with the target anatomical region. At least one of the first portion or the second portion being configured to facilitate in situ manipulation of the medium via an external manipulation system.

According to an embodiment, a medical device insertable within a target anatomical region includes a body, at least a first portion of the body being configured to be insertable within a target anatomical region, at least a second portion of the body being configured to facilitate retention of the body in the target anatomical region, and a connecting portion for facilitating a removable connection between the body and a delivery system for delivering a medium.

According to an embodiment, a medical device includes a body extending from a proximal end to a distal end, a connecting portion connected to the body at the proximal end, and a portion formed on the body adjacent to the distal end that can be configured to receive a medium and deliver the medium to a body cavity.

According to an embodiment, a system includes a medical device and a delivery system comprising a connecting portion for removably connecting to a connecting portion of the medical device. The medical device includes a body, at least a first portion of the body being configured to be insertable within a target anatomical region, and at least a second portion of the body being configured to facilitate retention of the body in the target anatomical region. The delivery system facilitates transfer of a medium toward the medical device.

According to an embodiment, a system includes a medical device and a delivery device removably connectable to the medical device, the delivery device being configured to maintain the medical device in an insertion configuration during insertion of the medical device inside the target anatomical region. The medical device includes a body, at least a first portion of the body being configured to be insertable within a target anatomical region, and at least a second portion of the body being configured to facilitate retention of the body in the target anatomical region. The delivery device being further configured to decouple the medical device from the delivery system after insertion of the medical device in the target anatomical region.

According to an embodiment, a system includes a dispenser configured to receive a fluid, a delivery member with a proximal end attached to the dispenser and a distal end that includes a connecting member and a medical device that can be configured to be positioned in a body cavity. The medical device includes a body extending from a proximal end to a distal end, a connecting member connected to the body at the proximal end that can be configured to mate to the coupler of the delivery member, and a portion formed on the body adjacent to the distal end that can be configured to receive the fluid and deliver the fluid into a body cavity.

According to an embodiment, a system includes a medical device that can be configured to be positioned in a body cavity to deliver a medium to the body cavity, and an external device positioned adjacent to the medical device.

According to an embodiment, a method of inserting a medical device into a sinus cavity includes deploying a distal end of a delivery device into a nasal cavity, positioning the distal end of the delivery device near a sinus ostium, advancing a guide wire of the delivery device through the sinus ostium and into the sinus cavity, advancing a medical device over the guide wire into the sinus cavity, wherein the medical device includes a body with a connecting portion at a proximal end and a portion at a distal end, and removing the guide wire from the sinus cavity, wherein the medical device remains in place in the sinus cavity.

A method includes coupling a dispenser to a medical device positioned in a cavity, delivering a fluid to the medical device, wherein the medical device includes a body with a connecting portion at a proximal end and a portion at a distal end, delivering the fluid to the cavity through the medical device, and decoupling the dispenser from the medical device.

### BRIEF DESCRIPTION OF THE DRAWINGS

FIG. 1A is a front view of a human head showing paranasal sinuses.
FIG. 1B is cross-sectional view of a part of the human head showing the paranasal sinuses.
FIG. 2 is a perspective view of a first embodiment of a medical device with a retention portion.
FIG. 3A is a schematic view of the first embodiment of the medical device positioned in a sinus cavity.
FIG. 3B is a schematic view of the first embodiment of the medical device positioned in the sinus cavity with a delivery system coupled to the medical device.
FIG. 4 is a flow chart showing a method of implanting the medical device in the sinus cavity.
FIG. 5A is a perspective view of a delivery device.
FIG. 5B is a schematic view of the delivery device being positioned in a nasal cavity.
FIG. 5C is a schematic view of a guide wire of the delivery device being inserted into the sinus cavity.
FIG. 5D is a schematic view of the medical device being inserted into the sinus cavity.
FIG. 5E is a schematic view of the guide wire being removed from the sinus cavity.
FIG. 5F is a schematic view of the delivery device being removed from the nasal cavity.
FIG. 5G is a schematic view of the delivery system coupled to the medical device.
FIG. 6 is a perspective view of a second embodiment of a medical device with retention portion that can be widened.
FIG. 7 is a perspective view of a third embodiment of a medical device with a retention portion that is wave-shaped.
FIG. 8 is a perspective view of a fourth embodiment of a medical device with a retention portion that is coiled.
FIG. 9A is a perspective view of a fifth embodiment of a medical device with a retention portion including a weeping balloon.
FIG. 9B is a perspective view of the fifth embodiment of the medical device showing the retention portion with an enlarged shape.
FIG. 10 is a perspective view of a sixth embodiment of a medical device with a hydrophilic coating on a retention portion.
FIG. 11 is a perspective view of a seventh embodiment of a medical device with a hydrophobic coating and a hydrophilic coating on a retention portion.
FIG. 12A is a perspective view of an eighth embodiment of a medical device including a biodegradable foam on a retention portion.
FIG. 12B is a perspective view of the eighth embodiment of the medical device showing the biodegradable foam enlarged.
FIG. 13 is a perspective view of a ninth embodiment of the medical device including magnetic pores.
FIG. 14A is a schematic view of a tenth embodiment of medical device positioned in a sinus cavity.
FIG. 14B is a schematic view of a therapeutic fluid being delivered to the tenth embodiment of the medical device.
FIG. 14C is a schematic view of tenth embodiment of the medical device enlarged.
FIG. 15A is a perspective view of an eleventh embodiment of a medical device.
FIG. 15B is a schematic view of the eleventh embodiment of the medical device positioned in an ethmoid sinus.
FIG. 16 is a schematic view of a first embodiment of a delivery member.
FIG. 17A is a schematic view of a second embodiment of a delivery member with flaps in a closed position.
FIG. 17B is a schematic view of the second embodiment of the delivery member with flaps in an open position.
FIG. 18A is a side view of a syringe.
FIG. 18B is a perspective view of a gas cylinder.
FIG. 18C is a side view of a squeeze bottle.
FIG. 18D is a cross-sectional view of a hand-held dispenser with an actuator.
FIG. 19A is a schematic view of a gel liquid and/or a gel foam in a sinus cavity.
FIG. 19B is a schematic view of a scaffold in the sinus cavity.
FIG. 20A is a schematic view of a delivery member including particles.
FIG. 20B is a schematic view of the delivery member adjacent to an external magnet.
FIG. 20C is a schematic view of the delivery member adjacent to an external magnet.
FIG. 21A is a schematic view of a connecting portion on a medical device coupled to a connecting portion on a delivery member.
FIG. 21B is a schematic view of the connecting portion on the medical device being decoupled form the connection portion on the delivery member.

### DETAILED DESCRIPTION

A medical device for implanting in a body cavity (e.g., a sinus cavity, a vagina or other portion of the female reproductive system, or the like) can be disclosed. The medical device can include a body that can be positioned in the sinus cavity. The medical device can extend through the sinus ostium and into the nasal cavity. A proximal end of the medical device (a portion positioned in the nasal cavity after implantation) can include a connecting portion that can be configured to couple the medical device to a dispenser. In an embodiment, the connecting portion on the medical device can be configured to couple to a connecting portion on the dispenser and/or a delivery member attached to the dispenser. The dispenser and/or delivery member can be magnetically coupled to and decoupled from the medical device. The dispenser and/or delivery member can be coupled to the medical device to dispense a fluid into the medical device. The fluid can flow through the medical device into the sinus cavity, such as to treat the sinus cavity.

A distal portion of the medical device can form a portion of the medical device. The portion of the medical device can include one or more pores through which the fluid can flow. Alternatively or in addition to the pores, the portion of the medical device can include a material that absorbs the fluid. The fluid can elute from the pores and/or the material on the portion of the medical device.

The medical device can provide access to the body cavity (e.g., sinus cavity, ear canal, GI tract, oral cavity, vagina, or other portion of the female reproductive system) through a natural orifice (e.g., the nostril, nasal cavity, vagina, or the like). Accessing the medical device through the natural orifice allows for refilling of the medical device with a fluid as needed. This access can allow for periodic treatment of the body cavity for chronic conditions (for instance, CRS). Further, the medical device can allow a fluid to be eluted from the medical device over a period of time (e.g., seconds, minutes, hours, days, etc.).

Embodiments described below illustrate the exemplary use of the medical device and a delivery device for treating CRS.

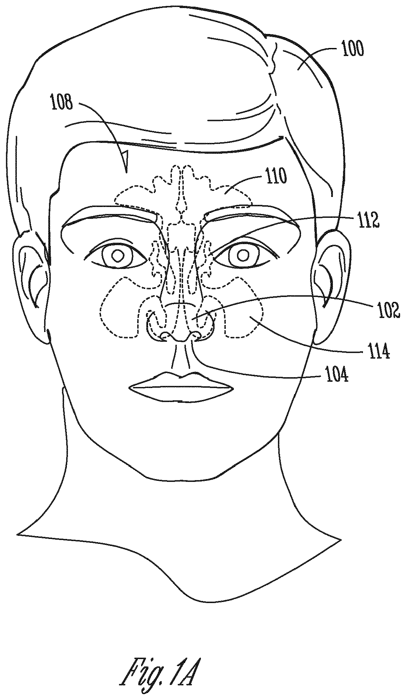

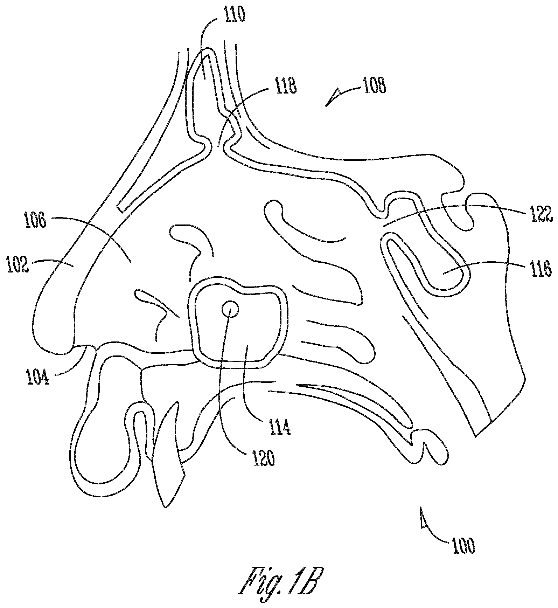

FIG. 1A can be a front view of human head 100 showing paranasal sinuses 102. FIG. 1B can be cross-sectional view of a part human head 100 showing paranasal sinuses 102. FIGS. 1A-1B show human head 100, nose 102, nostrils 104, nasal cavity 106, and paranasal sinuses 108. Paranasal sinuses 108 include frontal sinuses 110, ethmoid sinuses 112 (shown in FIG. 1A), maxillary sinuses 114, sphenoid sinuses 116 (shown in FIG. 1B), frontal sinus ostium 118 (shown in FIG. 1B), maxillary sinus ostium 120 (shown in FIG. 1B), and sphenoid sinus ostium 122 (shown in FIG. 1B).

Human head 100 can be shown in FIGS. 1A-1B. Human head 100 includes nose 102 positioned in a center of human head 100. Nose 102 has two nostrils 104 leading to nasal cavity 106. Paranasal sinuses 108 are positioned in human head 100 and are connected to nasal cavity 106. Paranasal sinuses 108 are a group of air-filled spaces in human head 100 that are positioned around nasal cavity 106.

Paranasal sinuses 108 include frontal sinuses 110, ethmoid sinuses 112, maxillary sinuses 114, and sphenoid sinuses 116. There are two frontal sinuses 110 positioned above each eye in the forehead region of human head 100. One frontal sinus ostium 118 connects each frontal sinus 110 to sinus cavity 106. There are two ethmoid sinuses 112 positioned between the eyes of human head 100. Ethmoid sinuses 112 each include several discrete air cells, also called Haller cells. There are two maxillary sinuses 114 positioned under each eye in the cheek region of human head 100. One maxillary sinus ostium 120 connects each maxillary sinus 114 to sinus cavity 106. There are two sphenoid sinuses 116 positioned behind each eye of human head 110. One sphenoid sinus ostium 122 connects each sphenoid sinus 116 to sinus cavity 106.

Paranasal sinuses 108 are normally filled with air. However, paranasal sinuses 108 are susceptible to becoming inflamed, which can block the sinus ostia and cause paranasal sinuses 108 to fill with fluid. Paranasal sinuses 108 can become blocked due for a number of different reasons, including infection, nasal polyps, a deviated nasal septum, chronic inflammation of the sinus ostium, and allergies, among others. Symptoms typically include nasal inflammation, anterior or posterior nasal discharge, nasal obstruction or congestion, loss of smell, and facial pain, tenderness, and swelling. These symptoms can persist for a period of time. Chronic rhinosinusitis (CRS) can be inflammation of paranasal sinuses 108 for a period lasting more than 12 weeks.

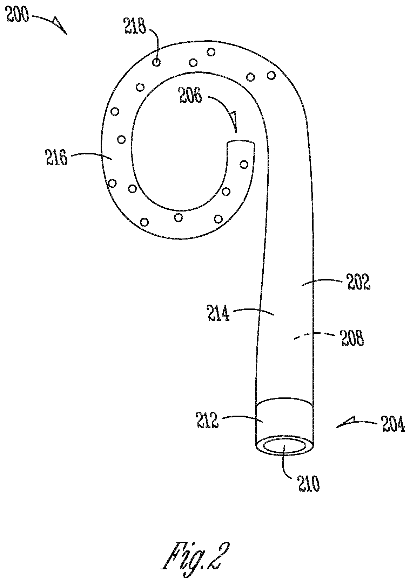

FIG. 2 can be a perspective view of medical device 200 with retention portion 216. Medical device 200, as illustrated, includes body 202, proximal end 204, distal end 206, lumen 208, opening 210, magnetic coupler 212, stem 214, retention portion 216, and pores 218.

Medical device 200 can be configured to be positioned and retained in a body cavity. In instances in which the medical device 200 can be configured for treatment of CRS, the body cavity can be a sinus cavity (e.g., maxillary, frontal, ethmoid, and the like). The medical device 200 can include body 202. In the embodiment shown in FIG. 2, the body 202 can be elongate and includes a proximal end 204 and distal end 206. The lumen 208 can extend from proximal end 204 to distal end 206 through body 202. The proximal end 204 can include the opening 210 to provide access to the lumen 208. The distal end 206 does, in the illustrated embodiment, does not include a through-flow lumen and can. Alternatively, body 202 is not be elongate, and can have a non-elongate shape.

In the embodiment shown in FIG. 2, the connecting portion 212 can be connected to the body 202 at the proximal end 204. The connecting portion 212 cancan cooperatively connect to a connecting portion of a dispenser (described elsewhere herein). In an embodiment, the connecting portion 212 on medical device 200 can include a magnetic material or a material that can be selectively magnetized (e.g., prior to or during one of the following: coupling of the medical device and the dispenser, placement of the medical device in the sinus cavity, delivery of a substance, and the like). Alternatively, other types of connections, such as frictional, mechanical couplers can be included with the medical device 200. Still further, the connecting portion 212 can be integral with the body.

In an embodiment, the retention portion 216 can be configured (e.g., shape, size, material properties) to retain the medical device 200 in the body cavity. Referencing FIG. 2, the retention portion 216 can be curled distally. In such embodiments, stem 214 forms a portion of the body 202 extending from the proximal end 204 to the retention portion 216. Curled retention portion 216 can extend from the stem 214 to the distal end 206. The curled retention portion 216 can include portion(s) of the medical device 200.

The body 202 of the medical device 200 can include a flexible, durable material. The body 202 of the medical device 200 can include a polymer material, for example polyurethane. Alternatively, the body 202 of the medical device 200 can include any suitable biocompatible material. In some aspects, the body 202 can include a shape memory material that can be pre-formed into a suitable shape to facilitate retention of the medical device 200 in the body cavity. In embodiments in which the body 202 includes a shape memory material, the body 202 can initially be in a shape suitable for insertion (e.g., elongate), and can return to a shape suitable for retention (e.g., curved with a preformed curve shape) after placement of the medical device 200 in the body cavity. In such embodiments, the body 202 can be elongate after a force can be applied or after guided by a guidewire during insertion. After the force or guidewire can be removed, the body 202 can revert to its preformed shape.

The retention portion 216 of medical device 200, as illustrated in FIG. 2, includes the pores 218. A fluid (e.g., a , cleansing, flushing, or other purpose fluid) can be inserted into the lumen 208 of the medical device 200 with a dispenser that can be coupled to medical device 200 at connecting portion 212. The fluid can move through the lumen 208 from the proximal end 204 to the distal end 206 and can move out of the pores 218 into the sinus cavity. Any number of the pores 218 can be included on the retention portion 216. The pores 218 can have any suitable shape and size, and the pores 218 can be distributed across the retention portion 216 in any suitable manner.

One or more fluids can be delivered to the medical device 200. The fluid(s) can include a liquid or a gas. The fluid can include any suitable fluid. For example, the fluid can include saline or air, such as for flushing mucus out of the sinus cavity, for aerating the sinus cavity, for cleansing the sinus cavity, for treating inflammation, and/or for lubricating dry sinus passages, or other cavity in which the medical device 200 can be inserted. The fluid can also be any suitable carrier medium, for example saline, air, or a cooling gas (such as nitrous oxide for mild sedation during treatment). The carrier medium can carry one or more beads, particles, disperse fibers, or suspensions, among others. The particles can include a pharmaceutical compound or can be radio-opaque or fluoresce under UV to aid visualization in addition to having properties. The pharmaceutical compound can include, for example, steroid (mometasone furoate) anti-microbial drugs, anti-inflammatories, or the like. The carrier medium can transport the particles into the sinus cavity for visualization and/or treatment. Further, the fluid can include pledget spheres, for example a gelatin foam coated with a drug or a radiopaque marker.

The pores 218 on retention portion 216 of the medical device 200 can be configured to release the fluid at a predetermined rate. For example, the pores 218 can be configured to elute the fluid into the sinus cavity over the course of about one to about ten days to treat the sinus cavity over that period of time.

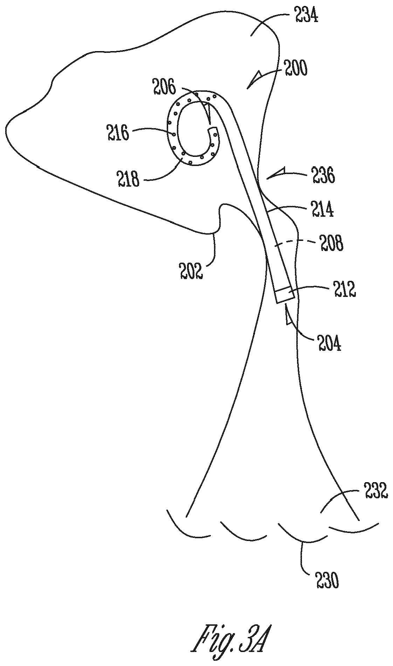

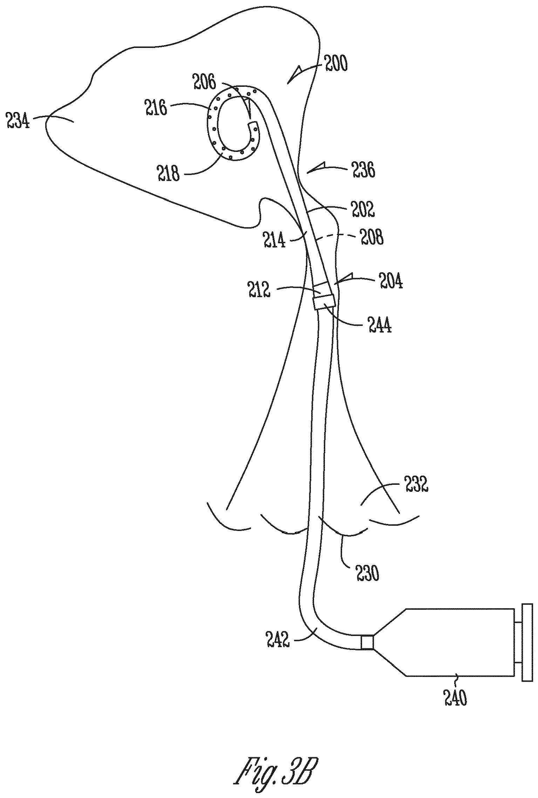

FIG. 3A can be a schematic view of the medical device 200 positioned in the sinus cavity 234. FIG. 3B can be a schematic view of the medical device 200 positioned in the sinus cavity 234 with a delivery system coupled to the medical device 200. The medical device 200, as illustrated, includes the body 202, the proximal end 204, the distal end 206, the lumen 208, the opening 210, the connecting portion 212, the stem 214, the retention portion 216, and the pores 218. FIGS. 3A-3B also show nostril 230, nasal cavity 232, sinus cavity 234, and sinus ostium 236. FIG. 3B also shows dispenser 240, delivery member 242, and connecting portion 244.

The medical device 200 can have the structure and configure as discussed above in reference to FIG. 2. FIGS. 3A-3B further show the nostril 230, the nasal cavity 232, the sinus cavity 234, and the sinus ostium 236. The nostril 230 can be an opening that leads to the nasal cavity 232. The sinus cavity 234 can be connected to the nasal cavity 232 through the sinus ostium 236. The sinus ostium 236 can be a passage connecting nasal cavity 232 to sinus cavity 234.

The retention portion 216 of the medical device 200 can be positioned in the sinus cavity 234 and can be configured to retain the medical device 200 in the sinus cavity 234. The retention portion 216 can be illustrated as having a curled shape in the embodiment shown in FIG. 2, but can have any shape and size that retains the medical device 200 in the sinus cavity 234. The stem 214 can extend away from the sinus cavity 234, through the sinus ostium 236, and into the nasal cavity 238. The connecting portion 212 of the medical device 200 can be positioned in the nasal cavity 232.

FIG. 3B shows the dispenser 240. The dispense can be positioned outside of the nostril 230. The dispenser 240 can be shown as being a syringe in FIG. 3B, but can be a gas cylinder, squeeze pump, hand-held dispenser with or without triggers, buttons or other actuators, or any other suitable dispenser in alternate embodiments. The delivery member 242 can be connected to the dispenser 240. The delivery member 242 can be integrally formed with the dispenser 240 or can be a separate piece that can be attached to the dispenser 240. The delivery member 242 and dispenser 240 can form a delivery system for delivering a medium (e.g., a fluid) to the medical device 200.

The delivery member 242 can include the connecting portion 244 positioned at a distal end. The Delivery member 242 can be configured to extend through the nostril 230 and into the nasal cavity 232, such as to couple with the medical device 200. In an embodiment, the connecting portion 244 on the delivery member 242 can be configured to magnetically couple with the connecting portion 212 on the medical device 200.

The connecting portion 212 on the medical device 200 can include a magnetic material or a magnetized material. The connecting portion 244 on the delivery member 242 can include a magnetic material or a magnetized material. If the connecting portion 212 on the medical device 200 includes a magnetic material, the connecting portion 244 on the delivery member 242 can include a magnetized material, such as to magnetically couple the connecting portion 212 and the connecting portion 244. Vice versa, if the connecting portion 244 on the delivery member 242 includes a magnetic material, the connecting portion 212 on medical device 200 can include a magnetized material to magnetically couple the connecting portion 212 and the connecting portion 244. One or more of the connecting portion 212 on the medical device 200 and the connecting portion 244 on the delivery member 242 can include a magnetized material. The connecting portion 244 and the connecting portion 212 can be decoupled by pulling them apart with a simple hand pull or separating them with a physical component. In an embodiment, the magnetic coupling of the connecting portion 212 and the connecting portion 244 can be strong enough to hold the connecting portion 212 and the connecting portion 244, and thus the medical device 200 and the delivery member 242, together as a fluid can be delivered to medical device 200, but weak enough to allow the connecting portion 212 and the connecting portion 244, and thus the medical device 200 and the delivery member 242, to be separated with a hand pull or a physical component without pulling the medical device 200 out of the sinus cavity 234.

The dispenser 240 can couple to the medical device 200, such as to deliver a fluid in the dispenser 240 to the medical device 200 through the delivery member 242. The connecting portion 212 on the medical device 200 and the connecting portion 244 on the delivery member 242 can provide for a releasable coupling of the medical device 200 and the dispenser 240. The medical device 200 can be reloaded with a fluid as often as needed. To reload the medical device 200, the connecting portion 244 on the delivery member 242 can be coupled with the connecting portion 212 on the medical device 200. A fluid can then be dispensed from the dispenser 240. The fluid can flow through the delivery member 242 into the medical device 200. The connecting portion 244 on the delivery member 242 can then be decoupled from the connecting portion 212 on the medical device 200 and the delivery member 242 can be removed from the nasal cavity 232.

The medical device 200 can provide a reloadable and reconnectable mechanism for delivering a fluid to the sinus cavity 232. The medical device 200 can be accessed via a natural orifice, such as the nostril 230, to be reloaded. After the medical device 200 has been positioned in the sinus cavity 232, the dispenser 240 can be coupled to the medical device 200 to refill the medical device 200 (as often as needed).

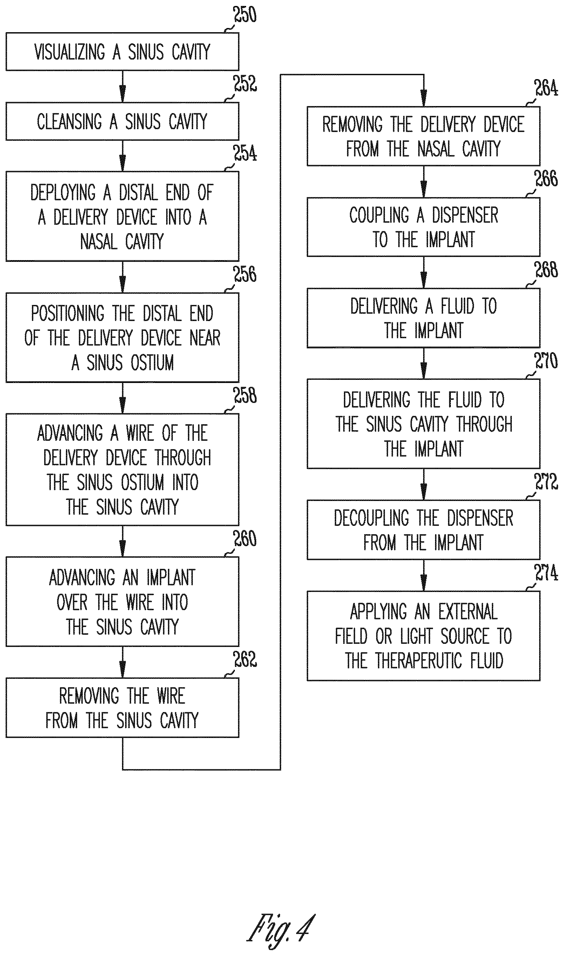

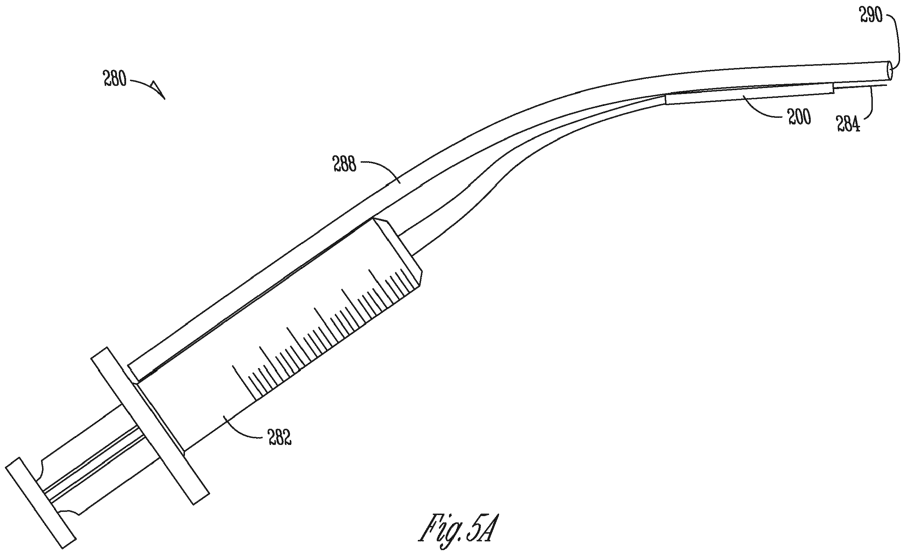

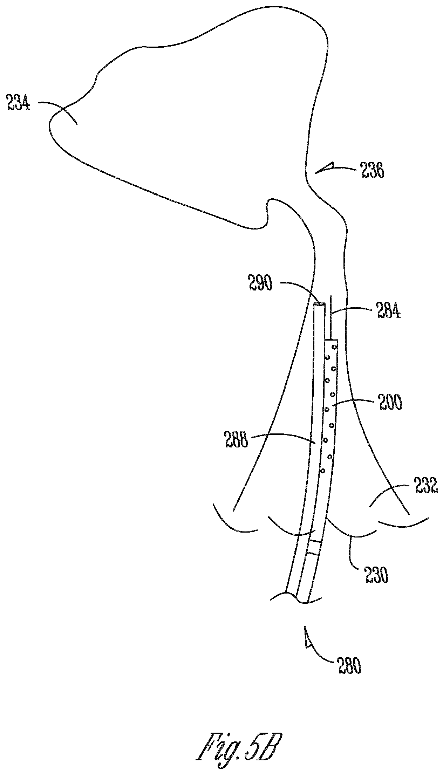

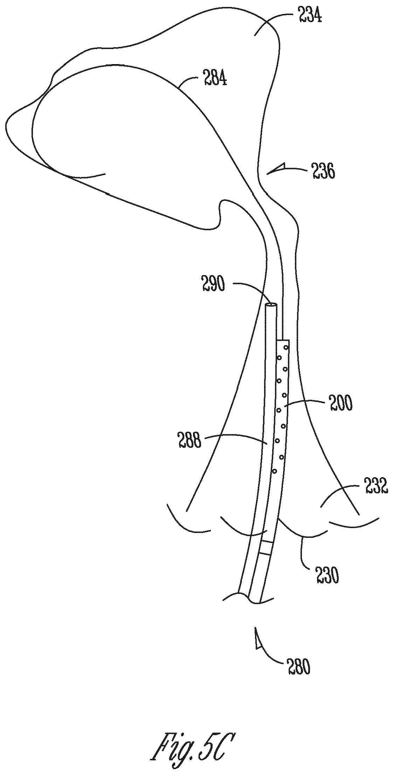

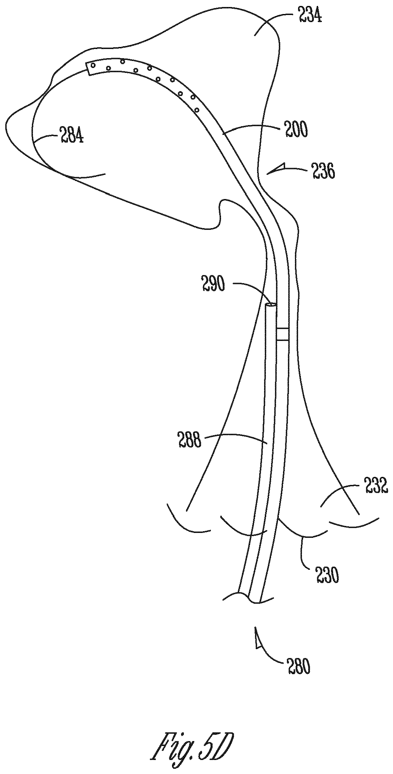

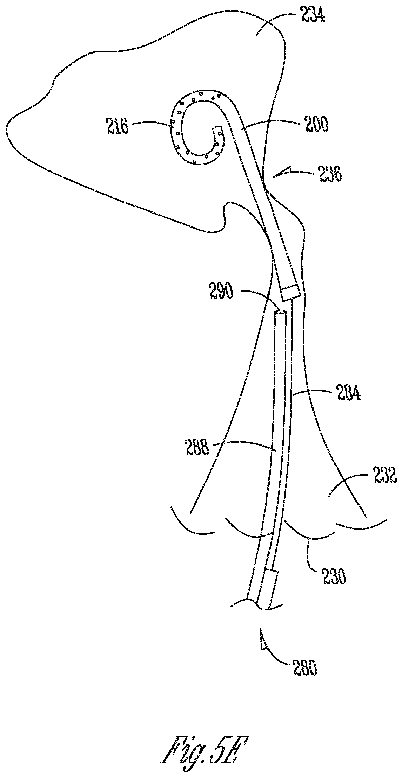

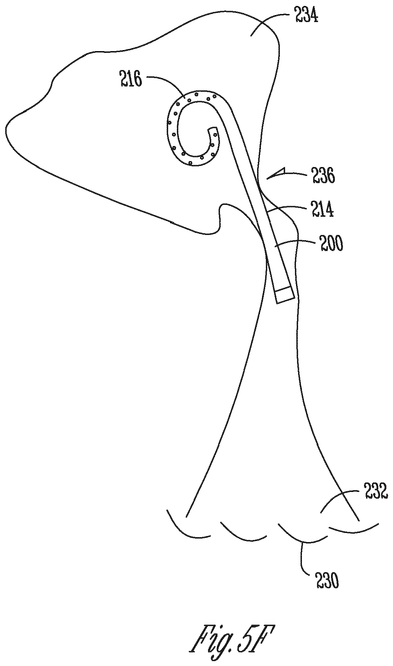

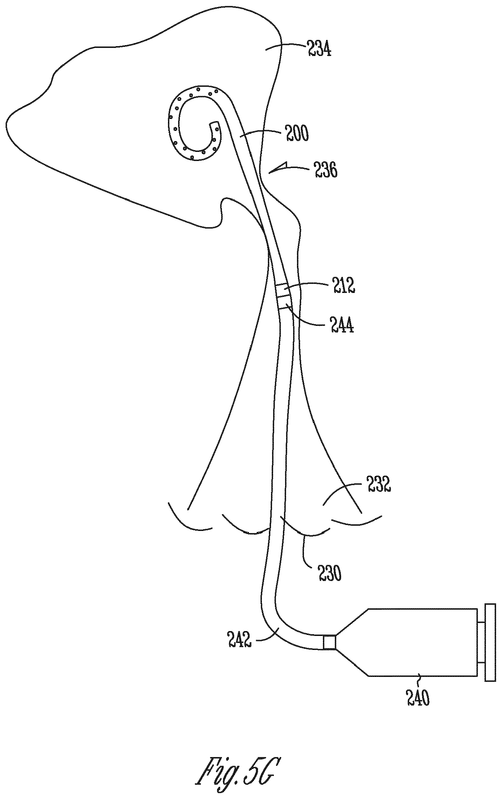

FIG. 4 can be a flow chart showing an example of a method of inserting the medical device 200 in the sinus cavity 234. FIG. 5A can be a perspective view of the delivery device 280 according to an embodiment. FIG. 5B can be a schematic view of the delivery device 280 being positioned in the nasal cavity 232. FIG. 5C can be a schematic view of a guide wire 284 of the delivery device 280 being inserted into the sinus cavity 234. FIG. 5D can be a schematic view of the medical device 200 being inserted into the sinus cavity 234. FIG. 5E can be a schematic view of the guide wire 284 being removed from the sinus cavity 234. FIG. 5F can be a schematic view of the delivery device 280 being removed from the nasal cavity 232. FIG. 5G can be a schematic view of a delivery system coupled to the medical device 200. FIGS. 4-5G can be discussed together. FIG. 4 includes operations 250-274.The medical device 200, as illustrated, includes the connecting portion 212, the stem 214, the retention portion 216, and the pores 218. The dispenser 240, as illustrated, includes the delivery member 242 and the connecting portion 244. The delivery device 280, as illustrated, includes the drug reservoir 282, the guide wire 284, the sheath 288, and a sensor 290 (e.g., a complementary metal oxide semiconductor (CMOS) or another sensor).

Some of the operations of FIG. 4 are optional. Operation 250 can include visualizing the sinus 234. An endoscope can be inserted in the nostril 230 to visualize the nasal cavity 232, the sinus cavity 234, and/or the sinus ostium 236, or other structure around the medical device 200. Alternatively, the sinus cavity 234 can be visualized externally using any suitable device.

Operation 252 can include cleansing the sinus cavity 234. The sinus cavity 234 can be cleansed by flushing a cleansing agent (e.g., saline or air) into the sinus cavity 234. In an embodiment, the cleansing can be performed by delivering the cleansing agent via a working channel of an endoscope. Additionally, or alternatively, any suitable cleaning device can be used to flush the cleansing agent into the sinus cavity 234. A lumen of the cleaning device can be inserted into the sinus cavity 234 and a cleansing agent can be applied to the sinus cavity 234 through the lumen. The cleansing agent can include a saline liquid or a gas that flows across the walls of the sinus cavity 234 to cleanse the sinus cavity 234. The cleansing agent can flow out of the sinus cavity 234, through the nasal cavity 232, and into a throat or out of a nose. Alternatively, a drain tube can be positioned around the lumen and the cleansing agent can flow out of sinus cavity 234 through the drain tube.

Operations 250 and 252 can be completed prior to inserting the medical device 200 into the sinus cavity 234. The operations 250 and 252 can be repeated (e.g., as needed for cleaning of the sinus cavity 234).

Operation 254 can include deploying a distal end of the delivery device 280 into the nasal cavity 232. In the embodiment shown in FIG. 5A, the delivery device 280 includes a drug reservoir 282. In an embodiment, the delivery device 280 does not include the drug reservoir 282. Further, in the embodiment shown in FIG. 5A, a nozzle extending from the drug reservoir 282 can be centered on the drug reservoir 282. In other embodiments, the nozzle can be offset from the center of the drug reservoir 282, can be angled with respect to the drug reservoir 282, and/or can be on an outside wall of the drug reservoir 282, such as for sensor attachment and manipulation during use. The guide wire 284 can extend from the delivery device 280. The medical device 200 can be positioned over the guide wire 284. The medical device 200 can be held sufficiently elongated for insertion by the guide wire 284. In the embodiment shown in FIG. 5A, the delivery device 280 further includes a sheath 288. The embodiment in FIG. 5A further includes a sensor 290 at a distal end of sheath 288. In an alternate embodiment, the delivery device 280 does not include the sheath 288 or the sensor 290. The sheath 288 can extend adjacent to the guide wire 284 to allow the sensor 290 to be more easily manipulated during use. The sensor 290 can be used to visualize the nasal cavity 232, the sinus cavity 234, and/or the sinus ostium 236, or other structure around the medical device 200.

Operation 256 can include positioning the distal end of the delivery device 280 near the sinus ostium 236, as shown in FIG. 5B. The sensor 290 can be used to help position the distal end of the delivery device 280 near sinus the ostium 236. Alternatively, external visualization (e.g., using an endoscope) can be used to help position the distal end of the delivery device 280 near the sinus ostium 236.

Operation 258 can include advancing the guide wire 284 of the delivery device 280 through the sinus ostium 236 and into the sinus cavity 234, as shown in FIG. 5C. The wire 284 can be configured to pass through the sinus ostium 236 even if the sinus ostium 236 can be inflamed or blocked.

Operation 260 can include advancing the medical device 200 over the guide wire 284 into the sinus cavity 234, as shown in FIG. 5D. The medical device 200 can be advanced into the sinus cavity 234 over the guide wire 284 using the delivery device 280. In embodiments, the cross-section of the medical device 200 can be sufficiently small to permit insertion through the sinus ostium 236 and into the sinus cavity 234, even in instances after the sinus ostium 236 can be at least partially blocked. Accordingly, the insertion procedure of the medical device 200 does not involve dilation of the sinus ostium 236, thereby reducing the chances of a patient experiencing pain during insertion.

Operation 262 can include removing the guide wire 284 from the sinus cavity 234, as shown in FIG. 5E. After the guide wire 284 can be removed from the sinus cavity 234, the medical device 200 cancan remain in the sinus cavity 234. The medical device 200 can be configured to be retained in the cavity upon removal of the guide wire 284. As described previously, various embodiments of the medical device 200 can include suitable materials (e.g., with shape memory alloys, or made of polymer, or another resilient material) and optionally formed with a preformed shape (e.g., curved, enlarged, etc.) upon removal of the guide wire 284. In the embodiment shown in FIG. 5E, the medical device 200 includes the retention portion 216 that cancan take its curled shape after the guide wire 284 can be removed, however, other alternatives are contemplated and described further below. After the guide wire 284 can be removed, the medical device 200 cancan be retained in the sinus cavity 234.

Operation 264 can include removing the delivery device 280 from the nasal cavity 232, as shown in FIG. 5F. After the delivery device 280 can be removed from the nasal cavity 232, the medical device 200 can decouple from delivery device 280. After the medical device 200 decouples from delivery device 200, the retention portion 216 of medical device 200 can remain positioned in the sinus cavity 234, and the stem 214 of the medical device 200 can extend from the sinus cavity 234, through the sinus ostium 236, and into the nasal cavity 232.

In one embodiment, medical device 200 can be preloaded with a fluid after it can be inserted into sinus cavity 234. In a second embodiment, drug reservoir 282 of delivery device 280 can be used to dispense a fluid into medical device 200 prior to removing guide wire 284 and delivery device 280 from sinus cavity 234 and nasal cavity 232. In a third embodiment, delivery device 280 can be removed from sinus cavity 234 and nasal cavity 232 and dispenser 240 can be coupled to medical device 200 to deliver a fluid to medical device 200.

In some embodiments, operations 266-272 discussed below can be completed after (e.g., immediately after) insertion of the medical device 200 into the sinus cavity 234, such as to deliver a fluid to the medical device 200. Alternatively,, operations 266-272 can be completed a period of time after the medical device 200 has been inserted into sinus cavity 234. Such embodiments can first aerate the sinus cavity 234 and, if it can be determined that a compound (e.g., saline, anti-inflammatory substance, etc.) can have to be delivered upon aeration, operations 266-272 can be completed after a period of time has passed following the placement of medical device 200. In other embodiments, operations 266-272 can be completed after placement of the medical device 200 to reload the medical device 200 with a fluid, such as to treat chronic conditions. Operations 266-272 can be repeated any number of times, (as needed) such as to deliver a fluid to sinus cavity 234.

Operation 266 can include coupling the dispenser 240 to the medical device 200, as shown in FIG. 5G. The dispenser 240 can include the delivery member 242 with the connecting portion 244 on a distal end of the delivery member 242. The delivery member 242 can be passed through the nostril 230 and into the nasal cavity 232 towards the medical device 200. The connecting portion 244 on the delivery member 242 can be magnetically coupled to the connecting portion 212 of the medical device 200, such as to couple the dispenser 240 to the medical device 200.

Operation 268 can include delivering a fluid to the medical device 200 using the dispenser 240. The dispenser 240 is shown as being a syringe in FIG. 5G, bu5G but any suitable dispenser in alternate embodiments. The fluid can be a cleansing agent used to cleanse the sinus cavity 234, including a saline or gas. The fluid can also be a therapeutic fluid, for example a therapeutic fluid including a therapeutic drug.

Operation 270 can include delivering the fluid in the medical device 200 to the sinus cavity 234. As discussed above in reference to FIG. 2, the medical device 200 can include the pores 218 through which the fluid can move to enter the sinus cavity 234. The medical device 200 can include any suitable number of the pores 218. The pores 218 can have any shape and size, and the pores 218 can positioned in any manner on the medical device 200.

Operation 272 can include decoupling the dispenser 240 from the medical device 200. The dispenser 240 can be decoupled from the medical device 200 by decoupling the connecting portion 244 of the delivery member 242 from the connecting portion 212 of the medical device 200. The connecting portion 244 and the connecting portion 212 can be decoupled by pulling them apart with a simple hand pull or separating them with a physical component.

Operation 274 can include applying an external field or a light source to the fluid. Operation 274 can be completed in conjunction with operation 270 while the fluid can be dispensed into the medical device 200 from the dispenser 240, or operation 274 can be completed after operation 272 after the dispenser 240 has been decoupled from the medical device 200. Operation 274 can include, for example, applying a magnetic field to the fluid as it can be delivered to medical device 200 to magnetize the fluid. Operation 274 can also include, for example, applying a UV light source to the fluid.

The medical device 200 can be removed from the sinus cavity 234. To remove the medical device 200 from the sinus cavity 234, a removal device with a connecting portion can be inserted into the nasal cavity 232. The removal device can couple to the connecting portion 212 on the medical device 200. For example, if the connecting portion 212 on the medical device 200 can be magnetic, the connecting portion on the removal device can be magnetic to couple to the connecting portion 212 on the medical device 200. The strength of the magnetic coupling can be strong enough to cause the medical device 200 to pull through the sinus ostium 236, the nasal cavity 232, and out of the nostril 230. Alternatively, a removal device with a wire could be inserted into the sinus cavity 234 to pull the medical device 200 out with the wire.

In embodiments, a patient interface can be provided, via software, such as for monitoring and tracking of symptoms prior to or after placement of the medical device 200. The patient can download a patient interface software, such as an app, to a mobile device that allows the patient and a medical provider to communicate. For example, a patient can log his/her symptoms in the app, such as pain level, inflammation, drainage, etc. Further, the app can prompt the patient to enter his/her symptoms. The interface can also prompt (either based on the recorded symptoms, predetermined visit schedule, or upon determination by a medical provider) the patient to visit in person with a medical provider. The medical provider can also ask questions to the patient through the app to follow up on the patient's symptoms and care.

Operations 250-274 discussed above are merely examples. Additional operations can be included, some operations can be excluded, and operations can be performed in different sequences. Further, the process for inserting the medical device 200 into each of the different paranasal sinuses can vary due to the placement of each of the paranasal sinuses.

The following discloses various embodiments of the medical device 200, the dispenser 240, and the delivery member 242. The following embodiments of the medical device 200 can be implanted and loaded with a fluid using the process described above. Further, any combinations of the medical devices, dispensers, and delivery members can be used. Further yet, the following discloses external fields that can be applied to any of the medical devices, dispensers, or delivery members.

Next, embodiments of medical devices are discussed. The medical devices can be used alternatively or in combination with embodiments of FIGS. 1-5G.

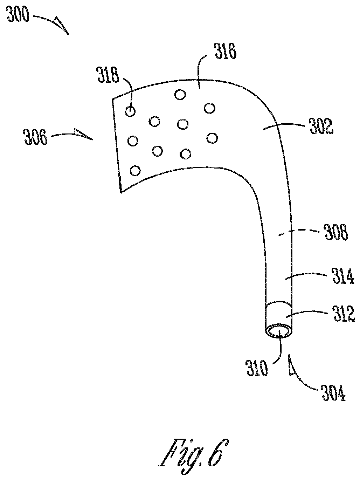

FIG. 6 can be a front view of a medical device 300 with a retention portion 316. The medical device 300 includes a body 302, a proximal end 304, a distal end 306, a lumen 308, an opening 310, a connecting portion 312, a stem 314, a retention portion 316, and pores 318.

The medical device 300 includes the body 302 having the proximal end 304 and the distal end 306. The lumen 308 can extend from the proximal end 304 to the distal end 306 through the body 302. The proximal end 304 can include the opening 310. The opening 310 can provide access to the lumen 308. The distal end 306, in some embodiments, does not include an opening that provides a through-flow for the lumen 308. The connecting portion 312 can be connected to the body 302 at the proximal end 304. The connecting portion 312 can be configured to connect to a connecting portion of a dispenser, such as to couple the medical device 300 to the dispenser, as discussed above in reference to FIGS. 2-5G. The stem 314 can form a portion of the body 302 extending from the proximal end 304 to the retention portion 316. The retention portion 316 can extend from the stem 314 to the distal end 306. The retention portion 316 can include portion(s) of the medical device 300. The retention portion 316 can be a widened portion in the embodiment shown in FIG. 6. The retention portion 316 can increase in cross-sectional area after placement in the cavity, such as to facilitate ease of insertion and retention of medical device 300. The increased cross-section of medical device 300 can be larger than the opening(s) in the sinus ostium.

After the medical device 300 can be positioned in a sinus cavity, the retention portion 316 can be positioned in the sinus cavity and the stem 314 can extend from the sinus cavity, through the sinus ostium, and into the nasal cavity. The retention portion 316 can be sized larger than the sinus ostium, such as to retain the medical device 300 in the sinus cavity. After a dispenser that has been magnetically coupled to medical device 300 (e.g., at the connecting portion 312) can be decoupled from medical device 300, the retention portion 316 can help retain the medical device 300 in the sinus cavity. Prior to placement of the medical device 300, the medical device 300 can have a cross-sectional area less than an opening size of a sinus ostium (e.g., with or without inflammation or blockage). Upon insertion into the sinus cavity (e.g., after removal of guide wire 284 as described above in reference to FIGS. 5A-5G), one or more portions of the medical device 300 can become larger than the opening size of the sinus ostium for retention of the medical device 300 in the sinus cavity. With reference to FIG. 6, the retention portion 316 can enlarge (e.g., become wider and/or thicker), such that the cross-section at the distal end 306 can be greater than the opening size of the sinus ostium. In some such embodiments, the cross-section at the distal end 306 can be greater than the cross-section at the proximal end 304.

The retention portion 316 of the medical device 300 can include the pores 318. A fluid can be delivered into the lumen 308 of the medical device 300 with a dispenser that can be coupled to the medical device 300. The fluid can move through the lumen 308 from the proximal end 304 toward the distal end 306 and can elute via the pores 318 into the sinus cavity. The pores 318 can also aerate the sinus cavity after no fluid can be delivered to the medical device 300. Any number of the pores 318 can be included on the retention portion 316. The pores 318 can have any suitable shape and size, and the pores 318 can be distributed across the retention portion 316 in any suitable manner.

The medical device 300 can be an alternate embodiment of the medical device 200 discussed above in reference to FIGS. 2-5G. The medical device 300 can be inserted into a sinus cavity using a similar process to the process discussed above in reference to FIGS. 4-5G. Further, any suitable dispenser can be coupled to the medical device 300, and any suitable fluid can be delivered to the medical device 300.

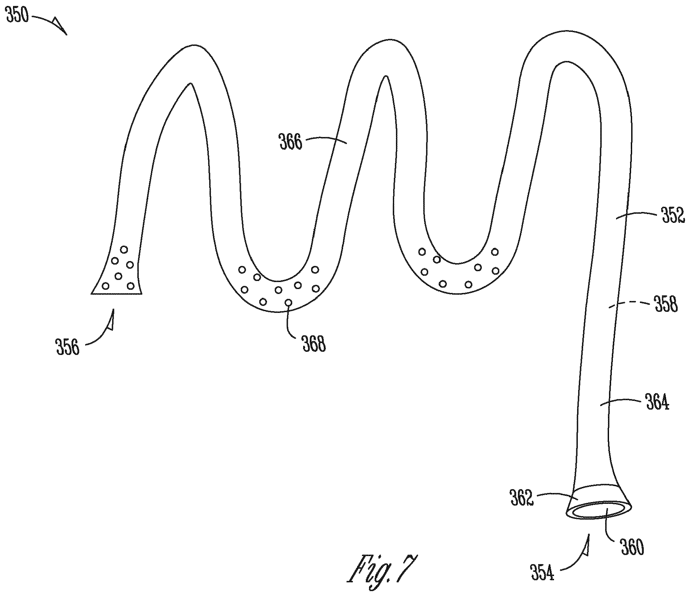

FIG. 7 can be a perspective view of medical device 350 with retention portion 366. The medical device 350 includes a body 352, a proximal end 354, a distal end 356, a lumen 358, an opening 360, a connecting portion 362, a stem 364, the retention portion 366, and pores 368.

The medical device 350 includes the body 352 having the proximal end 354 and the distal end 356. The lumen 358 can extend from the proximal end 354 to the distal end 356 through the body 352. The proximal end 354 can include the opening 360 to provide access to the lumen 358. The distal end 356, in some embodiments, does not include an opening that provides a through-flow for the lumen 358. The connecting portion 362 can be connected to the body 352 at the proximal end 354. The connecting portion 362 can be configured to connect to a connecting portion of a dispenser, such as to couple the medical device 350 to the dispenser, as discussed above in reference to FIGS. 2-5G. The stem 364 can form a portion of the body 352 extending from the proximal end 354 to the retention portion 366. The retention portion 366 can extend from the stem 364 to the distal end 356. The retention portion 366 can be a portion of the medical device 350.

The retention portion 366 can be wave-shaped in the embodiment shown in FIG. 7. The wave-shape includes a series of peaks and valleys. The retention portion 366 can have the same or similar cross-sectional area as a cross-sectional area of the stem 364. The retention portion 366 can be oriented at an angle to the proximal end 354, such as to retain the medical device 350 in the sinus cavity. After the medical device 350 can be positioned in a sinus cavity, the retention portion 366 can be positioned in the sinus cavity and the stem 364 can extend from the sinus cavity, through the sinus ostium, and into the nasal cavity. The retention portion 366 can be shaped to retain the medical device 350 in the sinus cavity. After a dispenser that has been magnetically coupled to the medical device 350 at the connecting portion 362 can be decoupled from the medical device 350, the retention portion 366 can help retain the medical device 350 in the sinus cavity. The retention portion 366 can be shown as having three peaks and two valleys in the embodiment shown in FIG. 7 but can include any number of peaks and valleys in alternate embodiments. The retention portion 366 can include a shape memory material, such as a polymer, that can be elongated while it can be being inserted into the sinus cavity and then resume its shape in the sinus cavity.

The retention portion 366 of the medical device 350 can include pores 368. In the embodiment shown in FIG. 7, the pores 368 can be positioned adjacent to the distal end 356 and on the two valleys. A fluid can be inserted into the lumen 358 of the medical device 350 with a dispenser that can be coupled to the medical device 350. The fluid can move through the lumen 358 from the proximal end 354 to the distal end 356 and can move out of the pores 368 into the sinus cavity. The pores 368 are positioned so that gravity can pull the fluid out of the pores 368. The pores 368 can aerate the sinus cavity after no fluid can be delivered to the medical device 350. Any number of pores 368 can be included on the retention portion 366. The pores 368 can have any suitable shape and size. The pores 368 can be distributed across the retention portion 366 in any suitable manner.

The medical device 350 can be an alternate embodiment of the medical device 200 discussed above in reference to FIGS. 2-5G. The medical device 350 can be inserted into a sinus cavity using a similar process to the process discussed above in reference to FIGS. 4-5G. Further, any suitable dispenser can be coupled to the medical device 350, and any suitable fluid can be delivered to the medical device 350.

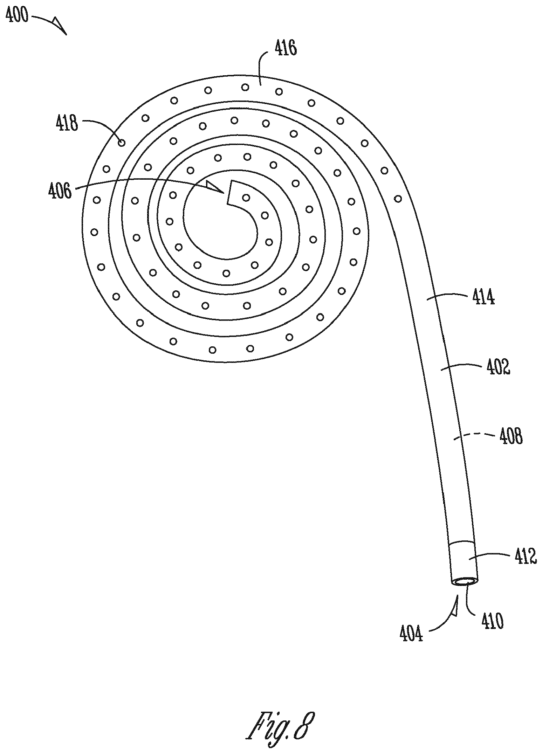

FIG. 8 can be a perspective view of a medical device 400 with a retention portion 516. The medical device 400, as illustrated, includes a body 402, a proximal end 404, a distal end 406, a lumen 408, an opening 410, a connecting portion 412, a stem 414, the retention portion 516, and pores 418.

The medical device 400 includes the body 402 having the proximal end 404 and the distal end 406. The lumen 408 can extend from the proximal end 404 to the distal end 406 through the body 402. The proximal end 404 can include the opening 410, such as to provide access to the lumen 408. The distal end 406, in some embodiments, does not include an opening that provides a through-flow for the lumen 408. The connecting portion 412 can be connected to the body 402 at the proximal end 404. The connecting portion 412 can be configured to connect to a connecting portion of a dispenser, such as to couple the medical device 400 to the dispenser, as discussed above in reference to FIGS. 2-5G. The stem 414 can form a portion of the body 402, The stem 414 can extend from the proximal end 404 to the retention portion 516. The retention portion 516 can extend from the stem 414 to the distal end 406. The retention portion 516 can be a portion of medical device 400.

The retention portion 516 has a coiled shape in the embodiment shown in FIG. 8. The coiled shape can be a spiral shape or a series of circles inside of one another and progressively getting smaller. After the medical device 400 is positioned in a sinus cavity, retention portion 516 can be positioned in the sinus cavity and the stem 414 can extend from the sinus cavity, through the sinus ostium, and into the nasal cavity. The retention portion 516 can be shaped to retain the medical device 400 in the sinus cavity. After a dispenser that has been magnetically coupled to medical device 400 at connecting portion 412 is decoupled from the medical device 400, the retention portion 516 can help retain the medical device 400 in the sinus cavity. The retention portion 516 is shown as being in a single plane in the embodiment shown in FIG. 8. In alternative embodiments, retention portion 516 can be shaped similarly to a spring. The retention portion 516 can include a shape memory material, such as a polymer, that can be held elongated while being inserted into the sinus cavity and then resume its shape in the sinus cavity.

The retention portion 516 of the medical device 400 can include the pores 418. A fluid can be inserted into the lumen 408 of the medical device 400 with a dispenser that can be coupled to the medical device 400. The fluid can move through the lumen 408 from the proximal end 404 to the distal end 406 and can move out of the pores 418 into the sinus cavity. The pores 408 can also aerate the sinus cavity after no fluid can be delivered to the medical device 400. Any number of the pores 418 can be included on enlarged portion 416. The pores 418 can have any suitable shape and size. The pores 418 can be distributed across the enlarged portion 416 in any suitable manner.

The retention portion 516 can contact walls of the sinus cavity after being positioned in the sinus cavity. A fluid can be absorbed directly into the walls of the sinus cavity through the pores 418, such as to directly treat the walls of the sinus cavity or the mucus membrane on the walls of the sinus cavity.

The medical device 400 can be an alternate embodiment of the medical device 200 discussed above in reference to FIGS. 2-5G. The medical device 400 can be inserted into a sinus cavity using a similar process to the process discussed above in reference to FIGS. 4-5G. Further, any suitable dispenser can be coupled to the medical device 400, and any suitable fluid can be delivered to the medical device 400.

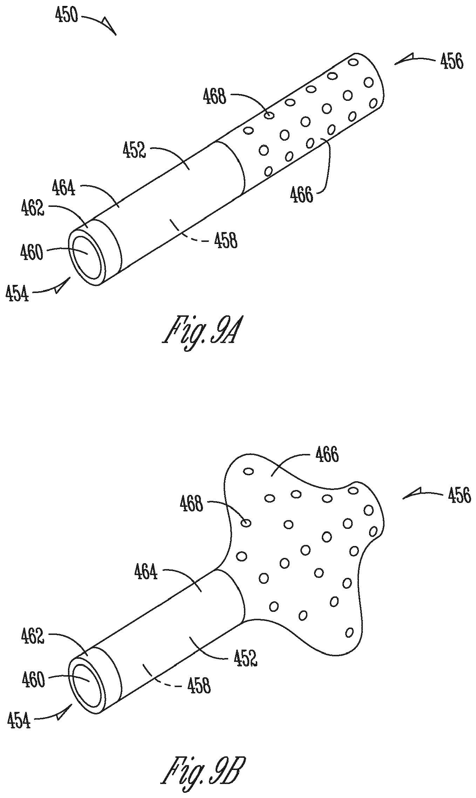

FIG. 9A is a perspective view of the medical device 450 including the retention portion 466. FIG. 9B is a perspective view of the medical device 400 showing the retention portion 466 with an enlarged shape. The medical device 450 can include a body 452, a proximal end 454, a distal end 456, a lumen 458, an opening 460, a connecting portion 462, a stem 464, the retention portion 466, and pores 468.

The medical device 450 can include the body 452. The body 452 can include the proximal end 454 and the distal end 456. The lumen 458 can extend from the proximal end 454 to the distal end 456 through the body 452. The proximal end 454 can include the opening 460, such as to provide access to the lumen 458. The distal end 456, in some embodiments, does not include an opening that provides a through-flow for the lumen 458. The connecting portion 462 can be connected to the body 452 at the proximal end 454. The connecting portion 462 can be configured to connect to a connecting portion of a dispenser, such as to couple the medical device 450 to the dispenser, as discussed above in reference to FIGS. 2-5G. The stem 464 can form a portion of the body 452 extending from the proximal end 454 to the retention portion 466. The retention portion 466 can extends from the stem 464 to the distal end 456. The retention portion 466 can include a portion of the medical device 450.

The retention portion 466 includes a weeping balloon in the embodiment shown in FIGS. 9A-9B. The weeping balloon can move between an original shape, as shown in FIG. 9A, and an enlarged shape, as shown in FIG. 9B. The retention portion 466, including the weeping balloon, of the medical device 450 can include pores 468. A fluid can be delivered to the medical device 450 through a dispenser that can be coupled to the medical device 450 at the connecting portion 462. The fluid can cause the retention portion 466 (e.g., the weeping balloon) to enlarge from its original shape, shown in FIG. 9A, to its enlarged shape, shown in FIG. 9B. After the retention portion 466 (e.g., the weeping balloon) can be enlarged, it can hold medical device 450 in position in the sinus cavity. The fluid can weep out of pores 468 into the sinus cavity. The pores 468 can be configured to allow the fluid to weep out over time. The pores 468 can aerate the sinus cavity after no fluid is being delivered to the medical device 450. Any number of pores 468 can be included on the retention portion 466. The pores 468 can have any suitable shape and size. The pores 568 can be distributed across the retention portion 466 in any suitable manner that allows the fluid to weep out of the retention portion 466. As the fluid weeps out of the pores 468, the retention portion 466 can deflate to its original position. The medical device 450 can be configured such that after the retention portion 466 (e.g., the weeping balloon) deflates to its original position, it can fall out of the sinus cavity.

The medical device 450 can be an alternate embodiment of the medical device 200 discussed above in reference to FIGS. 2-5G. The medical device 450 can be inserted into a sinus cavity using a similar process to the process discussed above in reference to FIGS. 4-5G. Further, any suitable dispenser can be coupled to the medical device 450, and any suitable fluid can be delivered to the medical device 450.

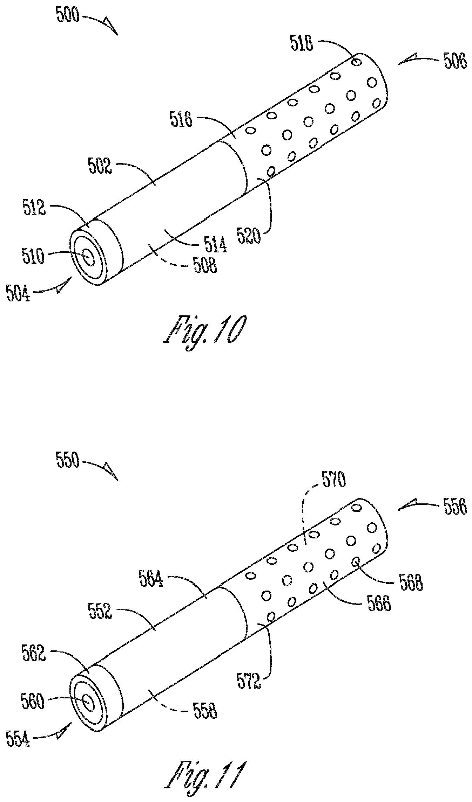

FIG. 10 is a perspective view of a medical device 500 with a hydrophilic coating 520 on a retention portion 516. The medical device 500 as illustrated includes a body 502, a proximal end 504, a distal end 506, a lumen 508, an opening 510, a connecting portion 512, a stem 514, the retention portion 516, pores 518, and the hydrophilic coating 520.

The medical device 500 as illustrated includes the body 502 having the proximal end 504 and the distal end 506. The lumen 508 can extend from the proximal end 504 to the distal end 506 through the body 502. The proximal end 504 can include the opening 510. The opening 510 can provide access to the lumen 508. The distal end 506, in some embodiments, does not include an opening that provides a through-flow for the lumen 508. The connecting portion 512 can be connected to the body 502 at the proximal end 504. The connecting portion 512 can be configured to connect to a connecting portion of a dispenser to couple the medical device 500 to the dispenser, as discussed above in reference to FIGS. 2-5G. The stem 514 can form a portion of the body 502 extending from the proximal end 504 to the retention portion 516. The retention portion 516 can extend from the stem 514 to the distal end 506. The retention portion 516 can include a therapeutic portion of medical device 500.

The retention portion 516 is shown as being substantially cylindrical in the embodiment shown in FIG. 10, but it can have any suitable shape in alternate embodiments. The retention portion 516 can help retain the medical device 500 in the sinus cavity. The retention portion 516 of the medical device 500 include pores 518. The medical device 500 as illustrated further includes the hydrophilic coating 520 positioned on the retention portion 516. The pores 518 can extend through the hydrophilic coating 520. After a fluid is delivered to the medical device 500, the fluid can flow through the pores 518 and be absorbed by the hydrophilic coating 520. The hydrophilic coating 520 can swell with the fluid. Hydrophilic coating 520 and fluid can be designed so that the hydrophilic coating 520 elutes the fluid at a predetermined rate. This can allow an agent suspended in the fluid to be administered to the sinus cavity over a period of time. The pores 518 can aerate the sinus cavity after fluid is eluted from the medical device 500. Any number of pores 518 can be included on the retention portion 516. The pores 518 can have any suitable shape and size. The pores 518 can be distributed across the retention portion 516 in any suitable manner.

The medical device 500 can be an alternate embodiment of the medical device 200 discussed above in reference to FIGS. 2-5G. The medical device 500 can be inserted into a sinus cavity using a similar process to the process discussed above in reference to FIGS. 4-5G. Further, any suitable dispenser can be coupled to the medical device 500, and any suitable fluid can be delivered to the medical device 500.

FIG. 11 is a perspective view of a medical device 550 with a hydrophobic coating 572 and a hydrophilic coating 570 on a retention portion 566. The medical device 550 as illustrated includes a body 552, a proximal end 554, a distal end 556, a lumen 558, an opening 560, a connecting portion 562, a stem 564, the retention portion 566, pores 568, the hydrophilic coating 570, and the hydrophobic coating 572.

The medical device 550 includes the body 552 having the proximal end 554 and the distal end 556. The lumen 558 extends from the proximal end 554 to the distal end 556 through the body 552. The proximal end 554 includes the opening 560, such as to provide access to the lumen 558. The distal end 556 does not include an opening that provides a through-flow for the lumen 558. The connecting portion 562 can be connected to the body 552 at the proximal end 554. The connecting portion 562 can be configured to connect to a connecting portion of a dispenser to couple the medical device 550 to the dispenser, as discussed above in reference to FIGS. 2-5G. The stem 564 can form a portion of the body 552. The stem 564 can extend from the proximal end 554 to the retention portion 566. The retention portion 566 can extend from the stem 564 to the distal end 556. The retention portion 566 can include a therapeutic portion of medical device 550.

The retention portion 566 is shown as being substantially cylindrical in the embodiment of FIG. 11, but it can have any suitable shape in alternate embodiments. The retention portion 566 can help retain the medical device 550 in the sinus cavity. The retention portion 566 of the medical device 550 can include the pores 568. The medical device 550 can further include the hydrophilic coating 570 positioned on the retention portion 566. The hydrophobic coating 572 can be positioned on the hydrophilic coating 570. The pores 568 can extend through the hydrophilic coating 570 and the hydrophobic coating 572. After a fluid is delivered to medical device 550, the fluid can flow through the pores 568 and be absorbed by hydrophilic coating 570. The hydrophilic coating 570 can swell with the fluid. The hydrophilic coating 570 and the fluid can be designed so that hydrophilic coating 520 elutes the fluid at a predetermined rate. This allows a therapeutic agent suspended in the fluid to be administered to the sinus cavity over a period of time. The hydrophobic coating 574 can be positioned over hydrophilic coating 572 to prevent hydrophilic coating 572 from absorbing fluids in the sinus cavity, such as the mucus in the sinus cavity. The pores 568 can aerate the sinus cavity fluid has been eluted by the medical device 550. Any number of pores 568 can be included on the retention portion 566. The pores 568 can have any suitable shape and size. The pores 568 can be distributed across retention portion 566 in any suitable manner.

The medical device 550 can be an alternate embodiment of the medical device 200 discussed above in reference to FIGS. 2-5G. The medical device 550 can be inserted into a sinus cavity using a similar process to the process discussed above in reference to FIGS. 4-5G. Further, any suitable dispenser can be coupled to the medical device 550, and any suitable fluid can be delivered to the medical device 550.

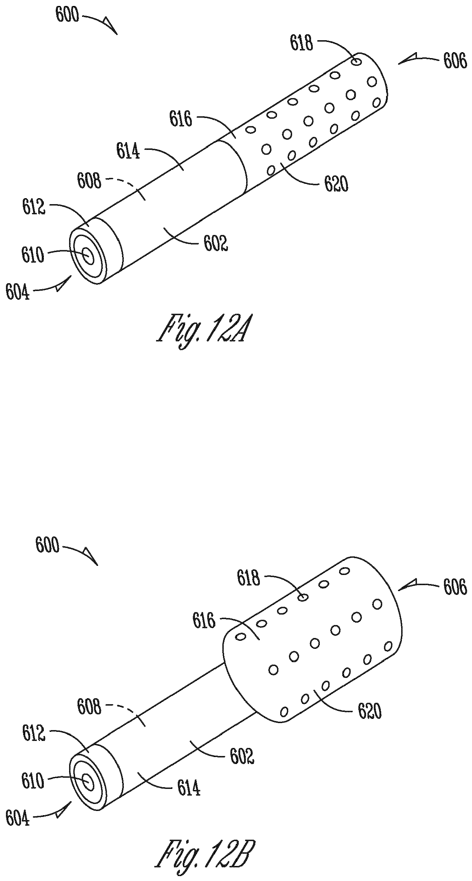

FIG. 12A is a perspective view of medical device 600 including a biodegradable foam 620 on a retention portion 616. FIG. 12B is a perspective view of the medical device 600 showing the biodegradable foam 620 enlarged. The medical device 600 as illustrated includes a body 602, a proximal end 604, a distal end 606, a lumen 608, an opening 610, a connecting portion 612, a stem 614, the retention portion 616, pores 618, and the biodegradable foam 620.

The medical device 600 includes the body 602 having the proximal end 604 and the distal end 606. The lumen 608 can extend from the proximal end 604 to the distal end 606 through the body 602. The proximal end 604 as illustrated includes the opening 610, such as to provide access to the lumen 608. The distal end 606, as illustrated, does not include an opening that provides a through-flow for the lumen 608. The connecting portion 612 can be connected to the body 602 at the proximal end 604. The connecting portion 612 can be configured to connect to a connecting portion of a dispenser, such as to couple medical device 600 to the dispenser, as discussed above in reference to FIGS. 2-5G. The stem 614 can form a portion of the body 602 that extends from the proximal end 604 to the retention portion 616. The retention portion 616 can extend from the stem 614 to the distal end 606. The retention portion 616 can include a therapeutic portion of medical device 600.

The retention portion 616 is shown as being substantially cylindrical in the embodiment shown in FIG. 12A, but it can have any suitable shape in alternate embodiments. The retention portion 616 can help retain the medical device 600 in the sinus cavity. The retention portion 616 of the medical device 600 can include the pores 618. The medical device 600 can include the biodegradable foam 620 positioned on the retention portion 616. After a fluid is delivered to medical device 600, the fluid can flow through the pores 618 and be absorbed by the biodegradable foam 620. The biodegradable foam 620 can swell from its original state, shown in FIG. 12A, to its enlarged state, shown in FIG. 12B, as it absorbs the fluid. The biodegradable foam 620 can then elute the agent over a period of time. The pores 618 can also aerate the sinus cavity after fluid delivered to the medical device 600. Any number of pores 618 can be included on the retention portion 616. The pores 618 can have any suitable shape and size. The pores 618 can be distributed across the retention portion 616 in any suitable manner.

The medical device 600 can be an alternate embodiment of the medical device 200 discussed above in reference to FIGS. 2-5G. The medical device 600 can be inserted into a sinus cavity using a similar process to the process discussed above in reference to FIGS. 4-5G. Further, any suitable dispenser can be coupled to the medical device 600, and any suitable fluid can be delivered to the medical device 600.

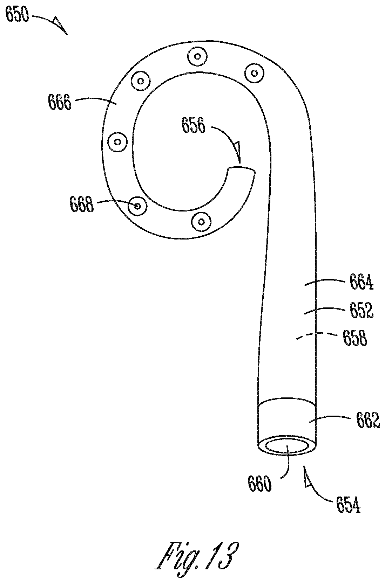

FIG. 13 is a perspective view of a medical device 650 including magnetic pores 668. The medical device 650 as illustrated includes a body 652, a proximal end 654, a distal end 656, a lumen 658, an opening 660, a connecting portion 662, a stem 664, a retention portion 666, and magnetic pores 668.

The medical device 650 includes the body 652 having the proximal end 654 and the distal end 656. The lumen 658 can extend from the proximal end 654 to the distal end 656 through the body 652. The proximal end 654 can include the opening 660 to provide access to lumen 658. The distal end 656, in some embodiments, does not includes an opening that provides a through-flow for the lumen 658. The connecting portion 662 can be connected to the body 652 at the proximal end 654. The connecting portion 662 can be configured to connect to a connecting portion of a dispenser, such as to couple the medical device 650 to the dispenser, as discussed above in reference to FIGS. 2-5G. The stem 664 can form a portion of the body 652. The stem 664 can extend from the proximal end 654 to the retention portion 666. The retention portion 666 can extend from the stem 664 to the distal end 656. The retention portion 666 can include a therapeutic portion of the medical device 650.

After the medical device 650 is positioned in a sinus cavity, the retention portion 666 can be positioned in the sinus cavity and the stem 664 extends from the sinus cavity, through the sinus ostium, and into the nasal cavity. The retention portion 666 is shown as having a curled shape in the embodiment shown in FIG. 13 but can have any suitable shape in alternate embodiments. The retention portion 666 can help retain the medical device 650 in the sinus cavity.

The retention portion 666 of the medical device 650 can include the magnetic pores 668. The magnetic pores 668 can include openings extending through the retention portion 666. The magnetic pores 668 can include a ring of magnetic material or magnetized material surrounding the opening. A fluid including magnetic spheres, which includes either a magnetic material or a magnetized material, can be inserted into the lumen 658 of the medical device 650, such as with a dispenser that can be coupled to the medical device 650. One or both of the magnetic pores 668 and/or magnetic spheres can include a magnetized material. The fluid can move through the lumen 658 from the proximal end 654 to the distal end 656 and can elute through the magnetic pores 668 into the sinus cavity. The magnetic spheres in the fluid can magnetically adhere to and hold in place on the magnetic pores 668. The pores 668 can aerate the sinus cavity before or after fluid eluted through the medical device 650. Any number of the magnetic pores 668 can be included on retention portion 666. The magnetic pores 668 can have any suitable shape and size. The magnetic pores 668 can be distributed across the retention portion 666 in any suitable manner.

The medical device 650 can be an alternate embodiment of the medical device 200 discussed above in reference to FIGS. 2-5G. The medical device 650 can be inserted into a sinus cavity using a similar process to the process discussed above in reference to FIGS. 4-5G. Further, any suitable dispenser can be coupled to the medical device 650, and any suitable fluid can be delivered to the medical device 650.

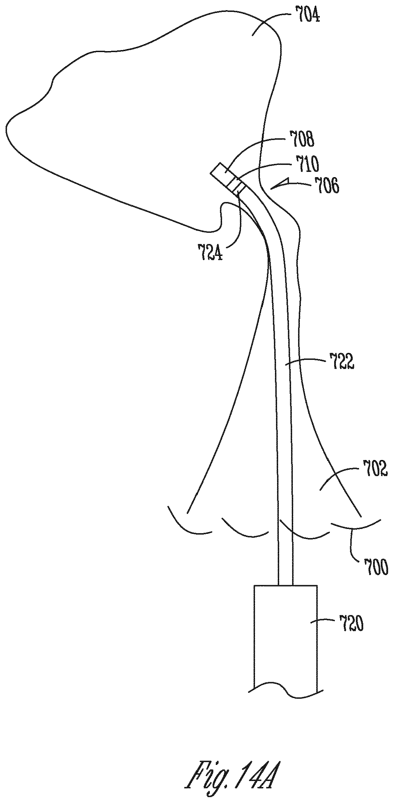

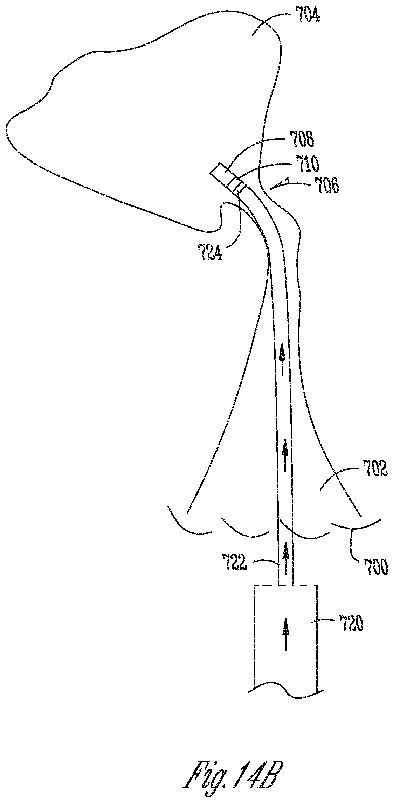

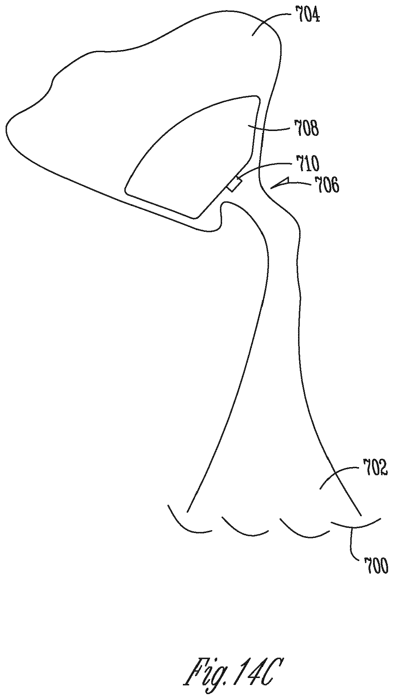

FIG. 14A is a schematic view of a medical device 708 positioned in a sinus cavity 704. FIG. 14B is a schematic view of a fluid being delivered to the medical device 708. FIG. 14C is a schematic view of the medical device 708 enlarged. FIGS. 14A-14C show a nostril 700, a nasal cavity 702, the sinus cavity 704, a sinus ostium 706, and the medical device 708. The medical device 708 can include a connecting portion 710. FIGS. 14A-14B further show a dispenser 720, a delivery member 722, and a connecting portion 724.

The nostril 700 is an opening that leads to nasal cavity 702. The sinus cavity 704 is connected to the nasal cavity 702 through the sinus ostium 706. The sinus ostium 706 is a narrow passage connecting nasal cavity 702 to sinus cavity 704.

The medical device 708 can include a pledget that can be positioned in the sinus cavity 704. A pledget can be a wad of absorbent material, such as a gelatin foam. The medical device 708 can include the connecting portion 710. In a first embodiment, the medical device 708 can be preloaded with a drug and/or radiopaque marker prior to being positioned in the sinus cavity 704. In an alternate embodiment, the medical device 708 can be positioned in the sinus cavity 704 and a fluid including a drug and/or radiopaque marker can be delivered to the medical device 708. As shown in FIG. 14A, the medical device 708 can be positioned in the sinus cavity 704 prior to being loaded with a drug and/or radiopaque marker. The dispenser 720, including the delivery member 722, can be positioned through the nasal cavity 702 to the medical device 708 in the sinus cavity 704. The delivery member 722 can include the connecting portion 724 that can magnetically couple to the connecting portion 710 on the medical device 708. As shown in FIG. 14B, a fluid in the dispenser 720 can be delivered to the medical device 708 through the delivery member 722. The medical device 708 can enlarge as it absorbs the fluid, as shown in FIG. 14C. After at least some of the fluid has been delivered to the medical device 708, the delivery member 722 can be removed.

As the medical device 708 expands, it can conform to the wall of the sinus cavity 704. The expanded medical device 708 can have a width that can be larger than the width of the sinus ostium 706. The expanded medical device 708 can be held in position in the sinus cavity 704. The medical device 708 can release the drug and/or radiopaque marker from the fluid at a predetermined rate based on the properties of the medical device 708 and the fluid.

The medical device 708 can be an alternate embodiment of the medical device 200 discussed above in reference to FIGS. 2-5G. The medical device 708 can be inserted into a sinus cavity using a similar process to the process discussed above in reference to FIGS. 4-5G. Further, any suitable dispenser can be coupled to the medical device 708, and any suitable fluid can be delivered to the medical device 708.

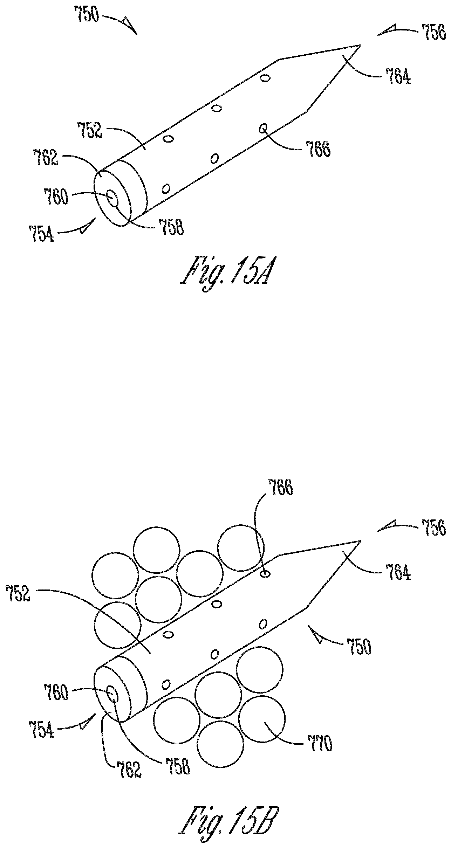

FIG. 15A is a perspective view of a medical device 750. FIG. 15B is a schematic view of the medical device 750 positioned in an ethmoid sinus 770. The medical device 750 as illustrated includes a body 752, a proximal end 754, a distal end 756, a lumen 758, an opening 760, a connecting portion 762, a tip 764, and openings 766. FIG. 15B also shows the ethmoid sinus 770.

The medical device 750 can include the body 752 having the proximal end 754 and the distal end 756. The medical device 750 can include a metallic material or a polymer material. The body 752 can be substantially rigid. The medical device 750 can be bioabsorbable. The lumen 758 can extend through the body 752 from the proximal end 754 to the distal end 756. The proximal end 754 can include the opening 750, such as to provide access to the lumen 758. The connecting portion 762 can be connected to the body 752 at the proximal end 754. The connecting portion 762 can be configured to connect to a connecting portion of a dispenser, such as to couple medical device 750 to the dispenser, as discussed above in reference to FIGS. 2-5G. The distal end 756 can include the tip 764. The tip 764 can include an atraumatic tip that can be used to push through tissue. The medical device 750 can include openings 766 extending through the body 752 to provide access to the lumen 758.

The medical device 750 can be inserted into the ethmoid sinus 770. The Ethmoid sinus 770 can be formed of air cells that are thin-walled cavities. The air cells of the ethmoid sinus 770 are formed of an air permeable membrane. After the ethmoid sinus 770 is infected, the air permeable membrane can clog. After the medical device 750 is inserted into the ethmoid sinus 770, the tip 774 can puncture through the air permeable membrane of numerous air cells forming the ethmoid sinus 770. Puncturing the air cells can help the air cells to drain. The openings 766 can help aerate the air cells. Further, a dispenser can then be coupled to the connecting portion 762 of the medical device 750. A fluid from the dispenser can flow through the opening 760 into the lumen 758 and then out through the openings 766 into the air cells. The fluid can include air that optionally includes a drug.

The medical device 750 can be an alternate embodiment of the medical device 200 discussed above in reference to FIGS. 2-5G. The medical device 750 can be inserted into a sinus cavity using a similar process to the process discussed above in reference to FIGS. 4-5G. Further, any suitable dispenser can be coupled to the medical device 750, and any suitable fluid can be delivered to the medical device 750.

Next, embodiments of delivery members are discussed. The delivery members can be used alternatively or in combination with embodiments of FIGS. 1-15.

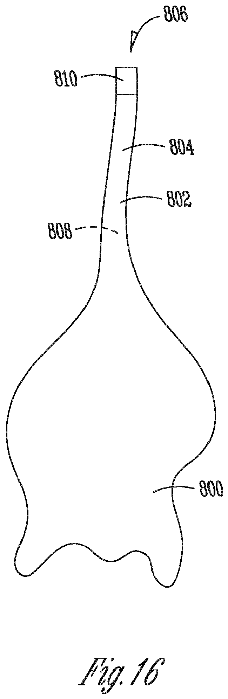

FIG. 16 is a schematic view of a delivery member 802. FIG. 16 shows a dispenser 800 and the delivery member 802. The delivery member 802 includes a body 804, a distal end 806, a lumen 808, and a connecting portion 810.

In the embodiment shown in FIG. 16, the dispenser 800 and the delivery member 802 are integrally formed. The delivery member 802 can be configured to extend between the dispenser 800 and a medical device that can be positioned in a sinus cavity or other cavity. The medical device can include any of the medical devices 200, 300, 350, 400, 450, 500, 550, 600, 650, 708, or 750 discussed above in reference to FIGS. 2-15B but can be any other suitable medical device in alternate embodiments.

The delivery member 802 can include the body 804 extending from the dispenser 800 to the distal end 806. The lumen 808 can be an opening that extends from the dispenser 800 to the distal end 806. The delivery member 802 can include the connecting portion 810 at the distal end 806 that can be configured to magnetically couple to a medical device. A fluid can flow from the dispenser 800, through the lumen 808 of the delivery member 802, and into the medical device.

The dispenser 800 and the delivery member 802 can include a resilient, flexible material. For example, the dispenser 800 and the delivery member 802 can be formed out of silicone or a rubber material. The dispenser 800 can be squeezed to dispense a fluid from the dispenser 800 through the delivery member 802 and into a medical device. The delivery member 802 can enlarge (e.g., temporarily) during delivery of the fluid to the medical device. The enlarged delivery member 802 can be positioned outside of the nasal cavity or in the nasal cavity. The enlarged delivery member 802 can be configured to not dilate a sinus ostium, the nasal cavity, or surrounding structures. The delivery member 802 can be configured to enlarge to allow for pressure control of the fluid in the delivery member 802 and to allow for better distribution of particles for periodic pressurization and delivery.

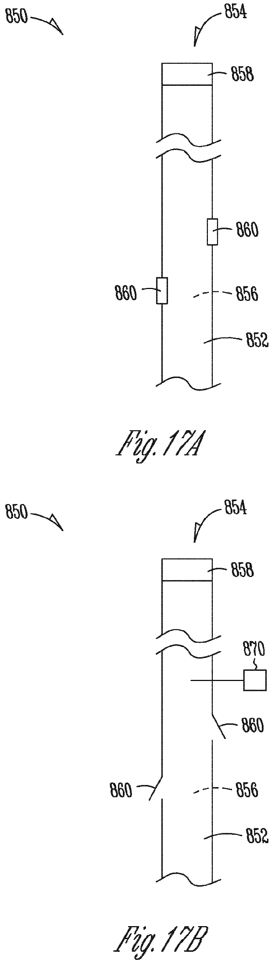

FIG. 17A is a schematic view of a delivery member 850 with outflow controls 860 in a closed position. FIG. 17B is a schematic view of the delivery member 850 with the outflow controls 860 in an open position. The delivery member 850 includes a body 852, a distal end 854, a lumen 856, a connecting portion 858, and outflow controls 860. FIG. 17B also shows a pressure sensor 870.

The delivery member 850 can be configured to extend between a dispenser and a medical device that can be positioned in a sinus cavity. The medical device can include any of the medical devices 200, 300, 350, 400, 450, 500, 550, 600, 650, 708, or 750 discussed above in reference to FIGS. 2-15B, but can be any other suitable medical devices in alternate embodiments.

The delivery member 850 can include the body 852 and can extend from a proximal end (not shown) to the distal end 854. The lumen 856 is an opening that can extend from the proximal end to distal end 854. The proximal end of delivery member 850 can be attached to a dispenser in any suitable manner. Alternatively, the delivery member 850 can be integrally formed with a dispenser. The delivery member 850 can include the connecting portion 858 at the distal end 854. The connecting portion 858 can be configured to magnetically couple to a medical device. A fluid can flow from a dispenser, through the lumen 856 of the delivery member 850, and into the medical device.

The outflow controls 860 can be positioned on the delivery member 850. The outflow controls 860 are flaps in the embodiment shown in FIGS. 17A-17B but can be any suitable mechanism in alternate embodiments. After the delivery member 850 is positioned in a nasal cavity, the outflow controls 860 can remain outside of the nasal cavity. In an unpressurized state, outflow controls 860 can be flush with delivery member 850, as shown in FIG. 17A. After the pressure in delivery member 850 rises above a threshold pressure level, the outflow controls 860 can move to an open position, as shown in FIG. 17B. The outflow controls 860 can open above a threshold pressure level, such as to relieve the pressure in the delivery member 850. The fluid in the delivery member 850 can leak out of the outflow controls 860 after outflow controls 860 are in an open position. The outflow can lower the pressure of the pressure in the delivery member 850. After the pressure has lowered below the threshold pressure level, the outflow controls 860 can close.

As shown in FIG. 17B, the pressure sensor 870 can be connected to the delivery member 850, such as to measure the pressure in the delivery member 850. The pressure in the delivery member 850 can indicate the pressure in the nasal cavity in which the delivery member 850 can be positioned. The nasal cavity can be very sensitive, so monitoring the pressure level in the nasal cavity can help to eliminate pain and discomfort during the procedure.

Next, embodiments of fluid dispensers are discussed. The dispensers can be used alternatively or in combination with embodiments of FIGS. 1-17B.

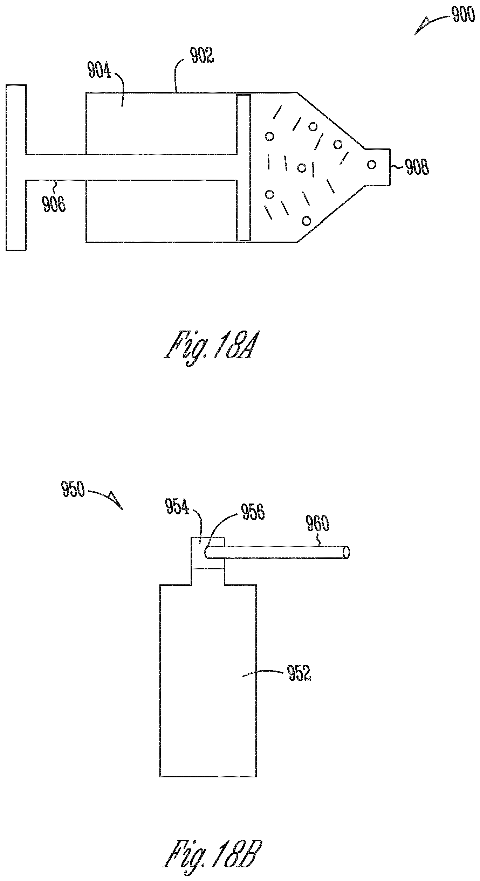

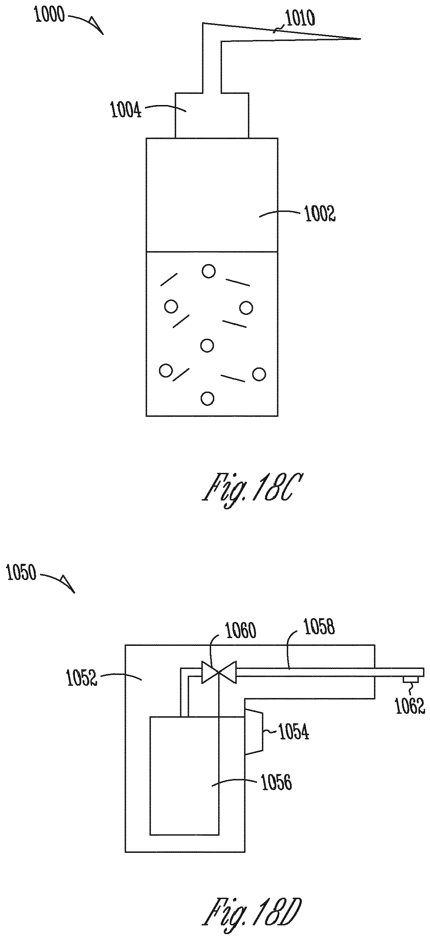

FIG. 18A is a side view of a syringe 900. FIG. 18B is a perspective view of a gas cylinder 950. FIG. 18C is a side view of a squeeze bottle 1000. FIG. 18D is a cross-sectional view of a dispenser 1050 with a solenoid valve 1060. FIG. 18A shows the syringe 900, which includes a body 902, a cavity 904, a plunger 906, and a coupler 908. FIG. 18B shows the gas cylinder 950, which includes a canister 952, a nozzle 954, and a coupler 956, and a delivery member 960. FIG. 18C shows the squeeze bottle 1000, which includes a bottle 1002, a cap 1004, and a delivery member 1010. FIG. 18D shows the hand-held dispenser 1050 having a body 1052, a trigger 1054, a reservoir 1056, a delivery member 1058, a solenoid valve 1060, and a sensor 1062.

The syringe 900 is shown in FIG. 18A. The syringe 900 can be any syringe known in the art. The syringe 900 can include the body 902 with the cavity 904. The cavity 904 can be configured to receive (via another component) a fluid. The plunger 906 can be positioned in the cavity 904. The coupler 908 can be positioned at a proximal end of the body 902. The body can include an opening extending from the cavity 904 and through the body 902. The coupler 908 can be configured to couple the syringe 900 to another device. For instance, the coupler 908 can be coupled to a delivery member, including any of the delivery members 800 or 850 discussed above in reference to FIGS. 16-17B, or to a medical device, including any of the medical devices 200, 300, 350, 400, 450, 500, 550, 600, 650, 708, or 750 discussed above in reference to FIGS. 2-15B. The plunger 906 can be configured to be pushed through the cavity 904 to push the fluid in the cavity 904 of out the syringe 900 through the opening at the coupler 908.

The gas cylinder 950 is shown in FIG. 18B. The gas cylinder 950 can be any gas cylinder known in the art. The gas cylinder 950 can include the canister 952. The canister can be configured to receive a gas. The nozzle 954 can be attached to the canister 952 and can include the coupler 956. The coupler 956 can be coupled to the delivery member 960. The delivery member 960 can be a rigid shaft. In alternative embodiments, the coupler 956 can be coupled to any delivery member, including any of the delivery members 800 or 850 discussed above in reference to FIGS. 16-17B, or to a medical device, including any of the medical devices 200, 300, 350, 400, 450, 500, 550, 600, 650, 708, or 750 discussed above in reference to FIGS. 2-15B. The nozzle 954 can be pressed downwards, such as to cause the gas in the canister 952 to flow through the nozzle 954 and the coupler 952 and out of the gas cylinder 950. The gas in the gas cylinder 950 can be pressurized prior to being loaded into the gas cylinder 950. Alternatively, the gas in the gas cylinder 950 can be pressurized with the nozzle 954.

The squeeze bottle 1000 is shown in FIG. 18C. The squeeze bottle 1000 can be any squeeze bottle known in the art. The squeeze bottle 1000 includes the bottle 1002 that can be configured to receive a fluid. The cap 1004 can be attached to the bottle 1002 and can be coupled to the delivery member 1010. The delivery member 1010 can be a rigid shaft. In alternative embodiments, the cap 1004 can be coupled to any delivery member, including any of the delivery members 800 or 850 discussed above in reference to FIGS. 16-17B, or to a medical device, including any of the medical devices 200, 300, 350, 400, 450, 500, 550, 600, 650, 708, or 750 discussed above in reference to FIGS. 2-15B. The bottle 1002 can be squeezed to push the fluid in the squeeze bottle 900 out of the squeeze bottle 1000 through the cap 1004.

The hand-held dispenser 1050 is shown in FIG. 18D. The hand-held dispenser 1050 can be any hand-held dispenser known in the art. The hand-held dispenser 1050 as illustrated includes the body 1052 that can be grasped by a user. The trigger 1054 can be positioned on the body 1052 so that a user's finger can rest on the trigger 1054 after holding the hand-held dispenser 1050. The reservoir 1056 can be positioned in the body 1052 and can be configured to hold a fluid. The delivery member 1058 has a proximal end that can be fluidly connected to the reservoir 1056. The delivery member 1058 can have a distal end positioned outside of the body 1052. The solenoid valve 1060 can be positioned in the delivery member 1058. The solenoid valve 1060 can be electrically connected to the trigger 1054. After a user presses trigger 1054, the solenoid valve 1060 can open, such as to allow the fluid in the reservoir 1056 to flow through the delivery member 1058 and outside of the hand-held dispenser 1050. After the trigger 1054 is released, the solenoid valve 1060 can block the delivery member 1058, such as to prevent the fluid from flowing out of the hand-held dispenser 1050. The solenoid valve 1060 can be any suitable actuator in alternate embodiments. The sensor 1062 can be positioned on the distal end of delivery member 1058. The sensor 1062 can be positioned outside of the body 1052. In alternate embodiments, the sensor 1062 can be positioned on the delivery member 1058 inside of the body 1052. The sensor 1062 can be a pressure or flow sensor to measure the pressure or flow of the fluid in the delivery member 1058.

The delivery member 1058 can be a rigid shaft. In alternate embodiments, the delivery member 1058 can have any suitable configuration, including any of the delivery members 800 or 850 discussed above in reference to FIGS. 16-17B. The distal end of the delivery member 1058 can optionally include a connecting portion to couple to a medical device, including any of the medical devices 200, 300, 350, 400, 450, 500, 550, 600, 650, 708, or 750 discussed above in reference to FIGS. 2-15B. Further, the distal end of the delivery member 1058 can be configured to connected to a second delivery member that can be configured to couple to a medical device.

The embodiments of the dispensers discussed above, including the syringe 900, the gas cylinder 950, the squeeze bottle 1000, and the hand-held dispenser 1050, are examples of dispensers and are not intended to be limiting. Any dispenser that can be capable of being coupled to a delivery member or a medical device can be suitable for use with any of medical devices 200, 300, 350, 400, 450, 500, 550, 600, 650, 708, or 750 discussed above in reference to FIGS. 2-15B.

Next, embodiments of external field systems are discussed. The external field systems can be used alternatively or in combination with embodiments of FIGS. 1-18D.

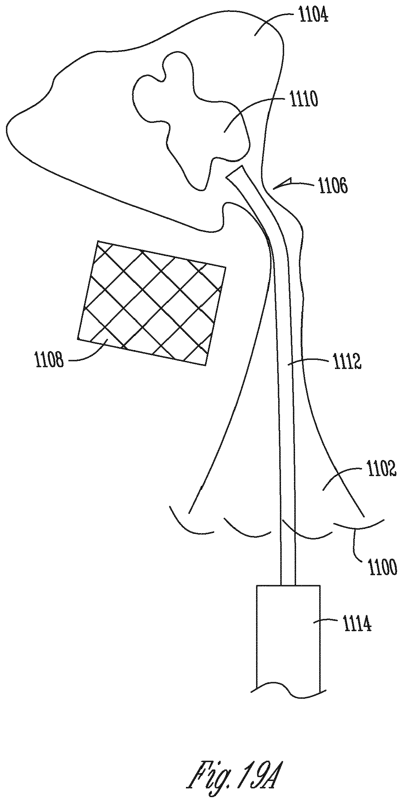

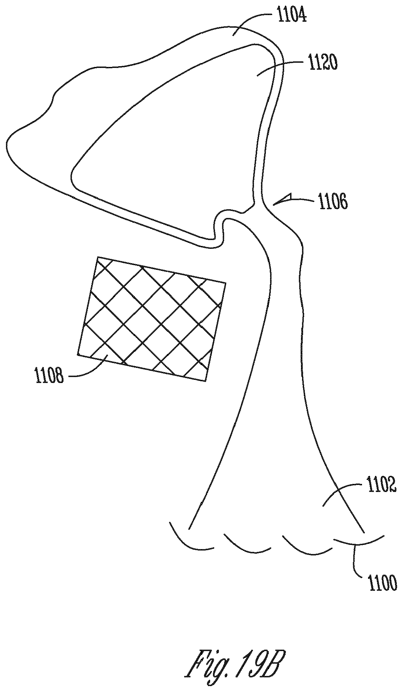

FIG. 19A is a schematic view of a gel liquid and/or a gel foam 1100 in a sinus cavity 1104. FIG. 19B is a schematic view of a scaffold 1120 in a sinus cavity 1104. FIGS. 19A-19B show a nostril 1100, a nasal cavity 1102, a sinus cavity 1104, a sinus ostium 1106, and a force transducer 1108. FIG. 19A further shows the gel liquid and/or the gel foam 1110, a delivery member 1112, and a dispenser 1114. FIG. 19B further shows the scaffold 1120.

The nostril 1100 is an opening that leads to nasal cavity 1102. The sinus cavity 1104 is connected to the nasal cavity 1102 through the sinus ostium 1106. The sinus ostium 1106 is a passage connecting the nasal cavity 1102 to the sinus cavity 1104.

FIGS. 19A-19B show an external manipulation system, including a force transducer 1108 positioned outside of and adjacent to the sinus cavity 1104. The force transducer 1108 can be positioned on the skin external to the nostril 1100. The force transducer 1108 can be an ultrasonic transducer, or other transducer. The gel liquid and/or the gel foam 1110 can be injected into the sinus cavity 1104 through the delivery member 1112. The gel liquid and/or the gel foam 1110 can include, for example, an open cell foam. The gel liquid and/or the gel foam 1110 can be a non-Newtonian fluid that can undergo shear thickening or shear thinning upon application of a force. A distal end of the delivery member 1112 can be positioned in the sinus cavity 1114. A proximal end of the delivery member 1112 can be connected to the dispenser 1114. The dispenser 1114 can inject the gel liquid and/or the gel foam 1110 into the sinus cavity 1104 through the delivery member 1102, as shown in FIG. 19A. After the gel liquid and/or the gel foam 1110 has been injected into the sinus cavity 1104, the delivery member 1112 and the dispenser 1114 can be removed from the sinus cavity 1104 and the nasal cavity 1102. The force transducer 1108 can be used to aid in the injection of the gel liquid and/or the gel foam 1110 into the sinus cavity 1104, such as by applying an external force field to the gel liquid and/or the gel foam 1110 to liquefy the gel liquid and/or the gel foam 1110 for transportation.

Force transducer 1108 can then apply a force field to gel liquid and/or gel foam 1110, which can undergo a transformation to scaffold 1120. Scaffold 1120 can have a semisolid or solid state. As the gel liquid and/or the gel foam 1110 undergoes the transformation to the scaffold 1120, it can expand to fill the sinus cavity 1104.

The gel liquid and/or the gel foam 1110 can include a drug suspended in it. The gel liquid and/or the gel foam 1110 can be injected in the sinus cavity 1104. After it undergoes the transformation to the scaffold 1120, it can start to elute the drug. Alternatively, a drug can be injected into the sinus cavity 1104 to be absorbed into the scaffold 1120 after the gel liquid and/or the gel foam 1110 has undergone the transformation.

The scaffold 1120 can contact one or more of the walls of the sinus cavity 1104, such as to help relieve sinus pressure. The scaffold 1120 can have a consistency that is about the same as the consistency of cartilage. The scaffold 1120 can biodegrade at a predetermined rate. Alternatively, the scaffold 1120 can contract as the drug is eluted. The scaffold 1120 can eventually fall out of the nostril 1100. The scaffold 1120 can be removed from the sinus cavity 1104, such as by using a vacuum removal tool that can be positioned in the nasal cavity 1102 and/or the sinus cavity 1104.

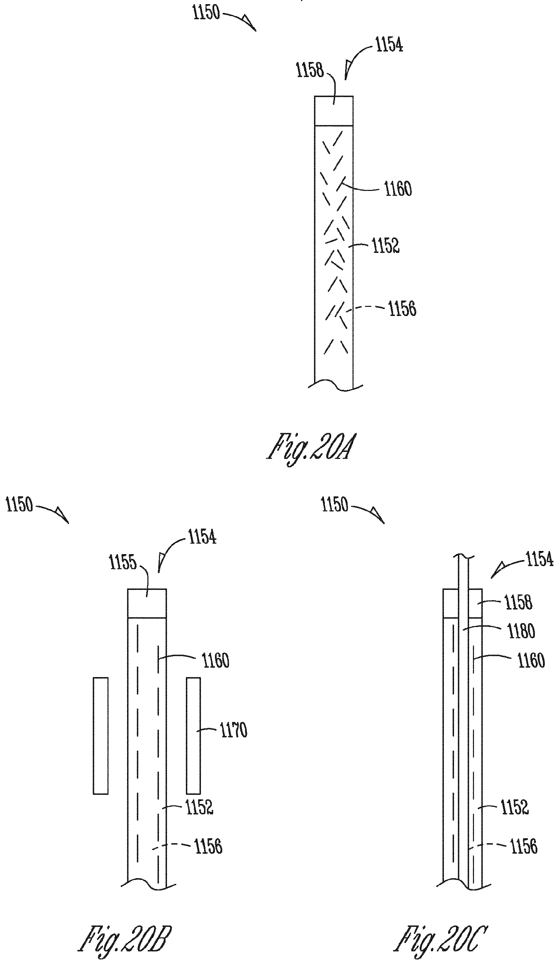

FIG. 20A is a schematic view of a delivery member 1150 including particles 1160. FIG. 20B is a schematic view of the delivery member 1150 adjacent to an external magnet 1170. FIG. 20C is a schematic view of the delivery member 1150 with a magnetic wire 1180 extending through the delivery member 1150. The delivery member 1150 as illustrated includes a body 1152, a distal end 1154, a lumen 1156, and a connecting portion 1158. FIGS. 20A-20C further show the particles 1160. FIG. 20B also shows the external magnet 1170. FIG. 20C also shows the magnetic wire 1180.

The delivery member 1150 can be configured to extend between a dispenser and a medical device that can be positioned in a sinus cavity. The medical device can include any of medical devices 200, 300, 350, 400, 450, 500, 550, 600, 650, 708, or 750 discussed above in reference to FIGS. 2-15B but can be any other suitable medical devices in alternate embodiments. The dispenser can include any dispenser, including the syringe 900, the gas cylinder 950, the squeeze bottle 1000, or the hand-held dispenser 1050 discussed above in reference to FIGS. 18A-18D.

The delivery member 1150 includes the body 1152. The body 1152 can extend from a proximal end (not shown) to the distal end 1154. The lumen 1156 can be an opening that extends from the proximal end to the distal end 1154. The proximal end of the delivery member 1150 can be attached to a dispenser in any suitable manner. Alternatively, the delivery member 1150 can be integrally formed with a dispenser. The delivery member 1150 can include the connecting portion 1158 at the distal end 1154. The connecting portion can be configured to magnetically couple to a connecting portion on a medical device. A fluid can flow from a dispenser, through the lumen 1156 of the delivery member 1150, and into the medical device.

The particles 1160 can be positioned in the delivery member 1160. The particles 1160 can include a magnetic material. As shown in FIG. 20A, the particles 1160 can be irregular with no magnetic field can be being applied thereto. As shown in FIGS. 20B-20C, the particles 1160 can align after an external manipulation system applies a magnetic field. FIG. 20B shows the external magnet 1170 positioned externally to delivery member 1150 and applying a magnetic field to the particles 1160. The magnet 1170 can magnetize the particles 1160. FIG. 20C shows the magnetic wire 1180 extending through the lumen 1156 of the delivery member 1150 and applying a magnetic field to the particles 1160, such as to magnetize the particles 1160.

The particles 1160 can include a drug with magnetic material attached to the drug. Some drugs are difficult to inject due to the composition and/or state of the drug. After a magnetic material is attached to the drug, a magnetic field can be applied to the particles 1160, as shown in FIGS. 20B-20C, such as to magnetize and align the particles 1160. Alignment between the particles can make it easier to inject the drug. After the particles 1160 are injected into a cavity, the magnetic field can be removed. Then the particles 1160 can be resume an irregular state in the sinus cavity. The drug can be retained in the sinus cavity in its irregular state.

Alternatively, the particles 1160 can be magnetized particles 1160 after they are held in a dispenser or the delivery member 1150. The particles 1160 can then be demagnetized prior to or after they are injected into the sinus cavity. For example, the particles 1160 can be magnetized particles that are attached to the magnetic wire 1180. If the magnetic wire 1180 can be demagnetized, the particles 1160 can be released from the magnetic wire 1180 and can be injected into the sinus cavity. The magnetic wire 1180 can also be magnetized again to attach more magnetized particles 1160 to it.

Next, embodiments of a connecting portion are discussed. The connecting portion can be used alternatively or in combination with embodiments of FIGS. 1-20B.

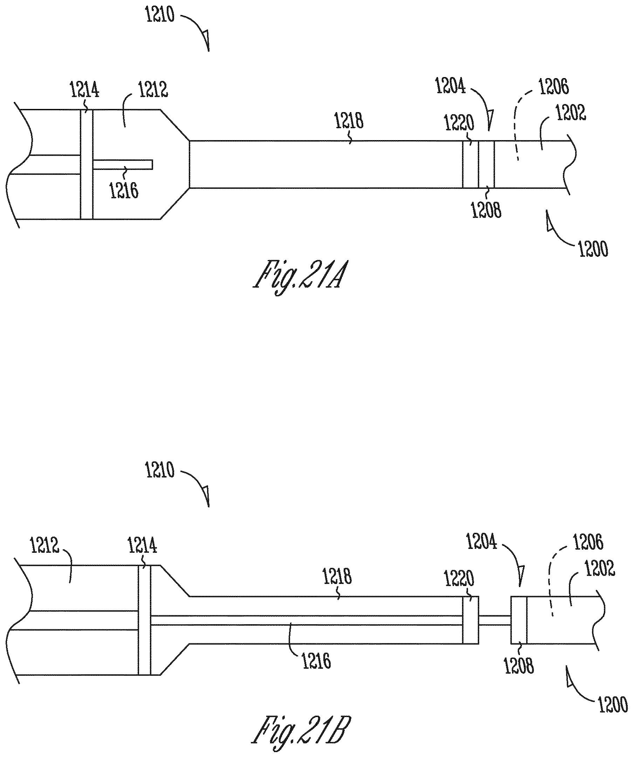

FIG. 21A is a schematic view of a connecting portion 1208 on a medical device 1200 coupled to a connecting portion 1220 on a delivery member 1218. FIG. 21B is a schematic view of the connecting portion 1208 on the medical device 1200 being decoupled from the connection portion 1220 on the delivery member 1218. The medical device 1200 as illustrated includes a body 1202, a proximal end 1204, a lumen 1205, and a connecting portion 1208. The delivery system 1210 as illustrated includes a dispenser 1212, a plunger 1214, a member 1216, the delivery member 1218, and the connecting portion 1220.

The medical device 1200 as illustrated includes the body 1202 extending from the proximal end 1204 to a distal end (not shown in FIGS. 21A-21B). The lumen 1206 can extend through the body 1202 of the medical device 1200 from the proximal end 1204 to the distal end. The connecting portion 1208 can be connected to the body 1202 at the proximal end 1204 of the medical device 1200.

The delivery system 1210 can include the dispenser 1212, which includes the plunger 1214 and the member 1216. The plunger 1214 can be positioned in the dispenser 1212. The member 1216, which can be a telescoping rod, can extend through the plunger 1214. The delivery system 1210 can include the delivery member 1218. The delivery member can extend from the dispenser 1212. The connecting member 1220 can be connected to a distal end of the delivery member 1218.

The connecting portion 1208 of the medical device 1200 and the connecting portion 1220 of the delivery member 1218 can include magnetic materials and can magnetically couple the medical device 1200 to the delivery member 1218, as shown in FIG. 21A. To decouple the medical device 1200 and the delivery member 1218, the member 1216 can telescope through the plunger 1214 and extend into the delivery member 1218. The member 1216 can be wider than an opening extending through the connecting portion 1208 of the medical device 1200, such as to push against the connecting portion 1208. This can decouple the connecting portion 1208 of the medical device 1200 and the connecting portion 1220 of the delivery member 1218, as shown in FIG. 21B.

The following are non-exclusive descriptions of possible embodiments of the present disclosure.

A medical device insertable within a target anatomical region includes a body, at least a first portion of the body being configured to be insertable within the target anatomical region, and at least a second portion of the body being configured to facilitate retention of the body in the target anatomical region. At least one of the first portion or the second portion being configured to interact with a portion surrounding the target anatomical region and/or configured to exchange a component to effect a change within the target anatomical region.

The medical device of the preceding paragraph can optionally include, additionally and/or alternatively, any one or more of the following features, configurations and/or additional components:

The body can, in some embodiments, be elongate and includes a proximal end and a distal end opposite to the proximal end. Alternatively, the body can include a non-elongate shape.

The first portion can be adjacent to the proximal end and the second portion can be adjacent to the distal end.

The body can include a connecting portion for facilitating connection to any of the following: delivery system, a dispenser, or one or more external actuators. In an embodiment, the connecting portion can be at the proximal end. Alternatively, the connecting portion can be positioned anywhere along the body, including at a distal end of the body.

In an embodiment, the connecting portion can advantageously permit ease of connection to a connector (e.g., a complementary connector) of an external component, including any of the following: delivery system, a dispenser, or one or more external actuators. The connecting portion can, advantageously self-seat, self-center, self-guide the connector of the external component positioned outside the body cavity. In an aspect, the connecting portion can connect to the connector of the external portion once the medical device has been inserted. Such embodiments can advantageously permit ease of connection, and/or repeated connection in the absence of visualization of the body cavity.

In an embodiment, the connecting portion can advantageously permit ease of removal of the connection to a connector (e.g., a complementary connector) of an external component. The connecting portion can include a removal mechanism that can facilitate removal of the connection between the connecting portion and the connector of the external component. Alternatively, the removal mechanism can be provided as a part of the external component.

In some embodiments, the connecting portion can include a magnetic component (e.g., permanent magnets, electromagnetic components, components that can selectively magnetize upon introduction of an external electric or magnetic field). Correspondingly, the external component can also include a magnetic component to facilitate ease of connection (e.g., in the absence of a visual feedback of the body cavity).

The magnetic connecting portion can be configured to couple to a magnetic connecting portion on a delivery system.

In certain embodiments, the body can include a lumen. In some such embodiments, the lumen can extend at least partially along portions of the body. In further embodiments, the lumen can extend from the proximal end of the body toward the distal end. In one aspect, the lumen does not extend through the entirety of the body. Accordingly, the distal end and the proximal end may not be in fluid communication with each other. Such embodiments can permit exchange of media with portions surrounding the body cavity. For example, in one instance, such an embodiment can facilitate aeration and/or gaseous exchange (e.g., via nasal cavity) to the exterior of the body. In another instance such an embodiment can facilitate transport and/or exchange of a medium (e.g., anti-inflammatory substance, mucus drainage, etc.) between the body cavity and an exterior of the body cavity. However, alternatives are contemplated, including the use of a lumen that runs substantially through the body (including through the distal end).

The body can include an opening. The lumen and the opening can in fluid communication with each other. Accordingly, the lumen can be configured to receive a medium through the opening.

The body can include pore(s) for facilitating exchange between the body cavity, via the medical device placed within the body cavity, and an exterior of the body cavity. In an embodiment, the pore(s) can be provided at least on the second portion of the body. The pore(s) can be distributed in accordance with desired rate of exchange of media (e.g., air, carbon dioxide, liquids, suspensions, particles, fibers, open or closed cell foams, etc.).For instance, if aeration can be desired, the pore(s) can facilitate exchange of air or carbon dioxide via the sinus ostia, nasal cavity, nostril(s) and to the exterior of the body. Alternatively, the pore(s) can transport, exchange, and/or elute component(s).

The pores are surrounded by a magnetic material.

As described previously, at least the second portion can be configured as a retention portion, to facilitate retention of the medical device inside the body cavity. For instance, in an embodiment, the second portion can have a cross-sectional area larger than an opening of the target anatomical region/body cavity. Alternatively, at least the second portion can pierce or anchor to portions of the target anatomical region/body cavity to facilitate retention of the medical device in the target anatomical region/body cavity.

In some embodiments, the second portion can have a cross-sectional area that can be larger than a cross-sectional area of the first portion.

The second portion has a cross-sectional area that can be larger than a cross-sectional area of an opening of the target anatomical region.

The second portion can be angled with respect to the first portion.

The second portion can have a curled shape, a widened shape, a coiled shape, or a wave shape.

At least a portion of the body can be coated with a hydrophilic coating.

The hydrophilic coating on a portion of the body can be coated with a hydrophobic coating.

The second portion can include a weeping balloon.

The weeping balloon can be configured to enlarge after filled with the fluid.

The second portion can include a foam and/or a pledget that can be configured to enlarge upon absorption of a medium.

The second portion can be or include a rigid member.

The second portion can be configured such that the medical device can anchor or pierce the Haller cells (ethmoid air cells). The second portion can be configured to be positioned in a frontal sinus, a maxillary sinus, or a sphenoid sinus.

A medical device insertable within a target anatomical region includes a body, at least a first portion of the body being configured to be insertable within a target anatomical region, and at least a second portion of the body being configured to facilitate retention of the body in the target anatomical region. At least one of the first portion or the second portion being configured to receive a medium and permit exchange thereof with the target anatomical region. At least one of the first portion or the second portion being configured to facilitate in situ manipulation of the medium via an external manipulation system.

The medical device of the preceding paragraph can optionally include, additionally and/or alternatively, any one or more of the following features, configurations and/or additional components:

The body can, in some embodiments, be elongate and include a proximal end and a distal end opposite to the proximal end. Alternatively, the body can include a non-elongate shape.

The first portion can be adjacent to the proximal end and the second portion can be adjacent to the distal end.

The body can include a connecting portion for facilitating connection to any of the following: delivery system, a dispenser, and/or one or more external actuators. In an embodiment, the connecting portion can be at the proximal end. Alternatively, the connecting portion can be positioned anywhere along the body, including at a distal end of the body.

In an embodiment, the connecting portion can advantageously permit ease of connection to a connector (e.g., a complementary connector) of an external component, including any of the following: delivery system, a dispenser, and/or one or more external actuators. The connecting portion can, advantageously self-seat, self-center, self-guide the connector of the external component positioned outside the body cavity. In an aspect, the connecting portion can connect to the connector of the external portion once the medical device has been inserted. Such embodiments can advantageously permit ease of connection, and/or repeated connection in the absence of visualization of the body cavity.

In an embodiment, the connecting portion can advantageously permit ease of removal of the connection to a connector (e.g., a complementary connector) of an external component. The connecting portion can include a removal mechanism that can facilitate removal of the connection between the connecting portion and the connector of the external component. Alternatively, the removal mechanism can be provided as a part of the external component.

In some embodiments, the connecting portion can include a magnetic component (e.g., permanent magnets, electromagnetic components, components that can selectively magnetize upon introduction of an external electric or magnetic field). Correspondingly, the external component can also include a magnetic component to facilitate ease of connection (e.g., in the absence of a visual feedback of the body cavity).

The magnetic connecting portion can be configured to couple to a magnetic connecting portion on a delivery system.

In certain embodiments, the body can include a lumen. In some such embodiments, the lumen can extend at least partially along portions of the body. In further embodiments, the lumen can extend from the proximal end of the body toward the distal end. In one aspect, the lumen does not extend through the entirety of the body. Accordingly, the distal end and the proximal end may not be in fluid communication with each other. Such embodiments can permit exchange of media with portions surrounding the body cavity. For example, in one instance, such an embodiment can facilitate aeration and/or gaseous exchange (e.g., via nasal cavity) to the exterior of the body. In another instance such an embodiment can facilitate transport and/or exchange of a medium (e.g., anti-inflammatory substance, mucus drainage, etc.) between the body cavity and an exterior of the body cavity. However, alternatives are contemplated, including the use of a lumen that runs substantially through the body (including through the distal end).

The body can include an opening. The lumen and the opening can be in fluid communication with each other. Accordingly, the lumen can be configured to receive a medium through the opening.

The body can include pore(s) for facilitating exchange between the body cavity, via the medical device placed within the body cavity, and an exterior of the body cavity. In an embodiment, the pore(s) can be provided at least on the second portion of the body. The pore(s) can be distributed in accordance with desired rate of exchange of media (e.g., air, carbon dioxide, liquids, suspensions, particles, fibers, open or closed cell foams, etc.). For instance, if aeration is desired, the pore(s) can facilitate exchange of air or carbon dioxide via the sinus ostia, nasal cavity, nostril(s) and to the exterior of the body. Alternatively, the pore(s) can transport, exchange, and/or elute component(s).

The pores can be surrounded by a magnetic material.

As described previously, at least the second portion can be configured as a retention portion, to facilitate retention of the medical device inside the body cavity. For instance, in an embodiment, the second portion can have a cross-sectional area larger than an opening of the target anatomical region/body cavity. Alternatively, at least the second portion can pierce or anchor to portions of the target anatomical region/body cavity to facilitate retention of the medical device in the target anatomical region/body cavity.

In some embodiments, the second portion can have a cross-sectional area that can be larger than a cross-sectional area of the first portion. The pores can be surrounded by a magnetic material. The second portion can be configured to be positioned in a frontal sinus, a maxillary sinus, or a sphenoid sinus.

A medical device insertable within a target anatomical region includes a body, at least a first portion of the body being configured to be insertable within a target anatomical region, at least a second portion of the body being configured to facilitate retention of the body in the target anatomical region, and a connecting portion for facilitating a removable connection between the body and a delivery system for delivering a medium.

The medical device of the preceding paragraph can optionally include, additionally and/or alternatively, any one or more of the following features, configurations and/or additional components:

The body can, in some embodiments, be elongate and include a proximal end and a distal end opposite to the proximal end. Alternatively, the body can include a non-elongate shape.

The first portion can be adjacent to the proximal end and the second portion can be adjacent to the distal end.

The body can include a connecting portion for facilitating connection to any of the following: delivery system, a dispenser, and/or one or more external actuators. In an embodiment, the connecting portion can be at the proximal end. Alternatively, the connecting portion can be positioned anywhere along the body, including at a distal end of the body.

In an embodiment, the connecting portion can advantageously permit ease of connection to a connector (e.g., a complementary connector) of an external component, including any of the following: delivery system, a dispenser, and/or one or more external actuators. The connecting portion can, advantageously self-seat, self-center, self-guide the connector of the external component positioned outside the body cavity. In an aspect, the connecting portion can connect to the connector of the external portion once the medical device has been inserted. Such embodiments can advantageously permit ease of connection, and/or repeated connection in the absence of visualization of the body cavity.

In an embodiment, the connecting portion can advantageously permit ease of removal of the connection to a connector (e.g., a complementary connector) of an external component. The connecting portion can include a removal mechanism that can facilitate removal of the connection between the connecting portion and the connector of the external component. Alternatively, the removal mechanism can be provided as a part of the external component.

In some embodiments, the connecting portion can include a magnetic component (e.g., permanent magnets, electromagnetic components, components that can selectively magnetize upon introduction of an external electric or magnetic field). Correspondingly, the external component can also include a magnetic component to facilitate ease of connection (e.g., in the absence of a visual feedback of the body cavity).

The magnetic connecting portion can be configured to couple to a magnetic connecting portion on a delivery system.

In certain embodiments, the body can include a lumen. In some such embodiments, the lumen can extend at least partially along portions of the body. In further embodiments, the lumen can extend from the proximal end of the body toward the distal end. In one aspect, the lumen does not extend through the entirety of the body. Accordingly, the distal end and the proximal end may not be in fluid communication with each other. Such embodiments can permit exchange of media with portions surrounding the body cavity. For example, in one instance, such an embodiment can facilitate aeration and/or gaseous exchange (e.g., via nasal cavity) to the exterior of the body. In another instance such an embodiment can facilitate transport and/or exchange of a medium (e.g., anti-inflammatory substance, mucus drainage, etc.) between the body cavity and an exterior of the body cavity. However, alternatives are contemplated, including the use of a lumen that runs substantially through the body (including through the distal end).

The body can include an opening. The lumen and the opening can be in fluid communication with each other. Accordingly, the lumen can be configured to receive a medium through the opening.

The body can include pore(s) for facilitating exchange between the body cavity, via the medical device placed within the body cavity, and an exterior of the body cavity. In an embodiment, the pore(s) can be provided at least on the second portion of the body. The pore(s) can be distributed in accordance with desired rate of exchange of media (e.g., air, carbon dioxide, liquids, suspensions, particles, fibers, open or closed cell foams, etc.). For instance, if aeration is desired, the pore(s) can facilitate exchange of air or carbon dioxide via the sinus ostia, nasal cavity, nostril(s) and to the exterior of the body. Alternatively, the pore(s) can transport, exchange, and/or elute component(s).

The pores can be surrounded by a magnetic material.

As described previously, at least the second portion can be configured as a retention portion, to facilitate retention of the medical device inside the body cavity. For instance, in an embodiment, the second portion can have a cross-sectional area larger than an opening of the target anatomical region/body cavity. Alternatively, at least the second portion can pierce or anchor to portions of the target anatomical region/body cavity to facilitate retention of the medical device in the target anatomical region/body cavity.

In some embodiments, the second portion can have a cross-sectional area that can be larger than a cross-sectional area of the first portion. The pores can be surrounded by a magnetic material.

A medical device insertable within a target anatomical region includes a body, at least a first portion of the body being configured to be insertable within the target anatomical region, and at least a second portion of the body being configured to facilitate retention of the body in the target anatomical region. At least one of the first portion or the second portion being configured to interact with a portion surrounding the target anatomical region and/or configured to exchange a component to effect a change within the target anatomical region.

The medical device of the preceding paragraph can optionally include, additionally and/or alternatively, any one or more of the following features, configurations and/or additional components:

The body can include a proximal end and a distal end.

The first portion can be adjacent to the proximal end and the second portion can be adjacent to the distal end.

The device can further include a connecting portion at a distal end of the body.

The connecting portion can be a magnetic connecting portion.

The magnetic connecting portion can be configured to couple to a magnetic connecting portion on a delivery system.

The device can further include a lumen extending through the body from a proximal end to a distal end; and an opening extending through the proximal end of the body to the lumen.

The lumen can be configured to receive a medium through the opening.

The device can further include pores on the second portion extending through the body.

The pores can bemagnetic pores, or can be surrounded by magnetic material.

The second portion can have a cross-sectional area that is larger than a cross-sectional area of the first portion.

The second portion can have a cross-sectional area that is larger than a cross-sectional area of an opening of the target anatomical region.

The second portion can be angled with respect to the first portion.

The second portion can have a curled shape, a widened shape, a coiled shape, or a wave shape.

At least a portion of the body can be coated with a hydrophilic coating.

The hydrophilic coating on a portion of the body can be coated with a hydrophobic coating.

The second portion can include a weeping balloon, or a balloon that is inflatable and/or deflatable.

The weeping balloon can be configured to enlarge when filled with the fluid.

The second portion can include a foam and/or a pledget that can be configured to enlarge upon absorption of a medium.

The second portion can include a rigid member.

The second portion can be configured such that the medical device can anchor or pierce the Haller cells (ethmoid air cells).

The second portion can be configured to be positioned in a frontal sinus, a maxillary sinus, or a sphenoid sinus.

A medical device insertable within a target anatomical region includes a body, at least a first portion of the body being configured to be insertable within a target anatomical region, and at least a second portion of the body being configured to facilitate retention of the body in the target anatomical region. At least one of the first portion or the second portion being configured to receive a medium and permit exchange thereof with the target anatomical region. At least one of the first portion or the second portion being configured to facilitate in situ manipulation of the medium via an external manipulation system.

The medical device of the preceding paragraph can optionally include, additionally and/or alternatively, any one or more of the following features, configurations and/or additional components:

The body can include a proximal end and a distal end.

The first portion can be adjacent to the proximal end and the second portion can be adjacent to the distal end.

The body can further include a connecting portion at a distal end of the body.

The connecting portion can be a magnetic connecting portion.

The magnetic connecting portion can be configured to couple to a magnetic connecting portion on a delivery system.

The body can further includie a lumen extending through the body from a proximal end to a distal end; and an opening extending through the proximal end of the body to the lumen.

The lumen can be configured to receive the medium through the opening.

The body can further include pores on the second portion extending through the body.

The pores can be magnetic pores, or can be surrounded by magnetic material.

The second portion can have a cross-sectional area that is larger than a cross-sectional area of the first portion.

The second portion can have a cross-sectional area that is larger than a cross-sectional area of an opening of the target anatomical region.

The second portion can be angled with respect to the first portion.

The second portion has a curled shape, a widened shape, a coiled shape, or a wave shape.

At least a portion of the body can be coated with a hydrophilic coating.

The hydrophilic coating on a portion of the body can be coated with a hydrophobic coating.

The second portion can include a weeping balloon, or a balloon that is inflatable and/or deflatable.

The weeping balloon can be configured to enlarge when filled with the fluid.

The second portion can include a foam and/or a pledget that can be configured to enlarge upon absorption of the medium.

The second portion can include a rigid member.

The second portion can be configured such that the medical device can anchor or pierce the Haller cells (ethmoid air cells).

The second portion can be configured to be positioned in a frontal sinus, a maxillary sinus, or a sphenoid sinus.

A medical device insertable within a target anatomical region includes a body, at least a first portion of the body being configured to be insertable within a target anatomical region, at least a second portion of the body being configured to facilitate retention of the body in the target anatomical region, and a connecting portion for facilitating a removable connection between the body and a delivery system for delivering a medium.

The medical device of the preceding paragraph can optionally include, additionally and/or alternatively, any one or more of the following features, configurations and/or additional components:

The body can include a proximal end and a distal end.

The first portion can be adjacent to the proximal end and the second portion can be adjacent to the distal end.

The connecting portion can be a magnetic connecting portion.

The magnetic connecting portion can be configured to couple to a magnetic connecting portion on the delivery system.

The body can further include a lumen extending through the body from a proximal end to a distal end; and an opening extending through the proximal end of the body to the lumen.

The lumen can be configured to receive the medium through the opening.

The body can further include pores on the second portion extending through the body.

The pores can be magnetic pores, or be surrounded by magnetic material.

The second portion can have a cross-sectional area that is larger than a cross-sectional area of the first portion.

The second portion can have a cross-sectional area that is larger than a cross-sectional area of an opening of the target anatomical region.

The second portion can be angled with respect to the first portion.

The second portion can have a curled shape, a widened shape, a coiled shape, or a wave shape.

At least a portion of the body can be coated with a hydrophilic coating.

The hydrophilic coating on a portion of the body can be coated with a hydrophobic coating.

The second portion can include a weeping balloon, or a balloon that is inflatable and/or deflatable.

The weeping balloon can be configured to enlarge when filled with the fluid.

The second portion can include a foam and/or a pledget that can be configured to enlarge upon absorption of the medium.

The second portion can include a rigid member.

The second portion can be configured such that the medical device can anchor or pierce the Haller cells (ethmoid air cells).

The second portion can be configured to be positioned in a frontal sinus, a maxillary sinus, or a sphenoid sinus.

A medical device includes a body extending from a proximal end to a distal end, a connecting portion connected to the body at the proximal end, and a portion formed on the body adjacent to the distal end that can be configured to receive a medium and deliver the medium to a body cavity.

The medical device of the preceding paragraph can optionally include, additionally and/or alternatively, any one or more of the following features, configurations and/or additional components:

The connecting portion can be a magnetic connecting portion.

The magnetic connecting portion can be configured to couple to a magnetic connecting portion on the delivery system.

The body can further include a lumen extending through the body from the proximal end to the distal end; and an opening extending through the proximal end of the body to the lumen.

The lumen can be configured to receive the medium through the opening.

The body can further include a stem extending from the proximal end to the portion.

The body can further include pores on the portion extending through the body.

The pores can surrounded by a magnetic material.

The portion can have a cross-sectional area that is larger than a cross-sectional area of a stem of the medical device.

The portion can have a cross-sectional area that is larger than a cross-sectional area of an opening of the body cavity.

The portion can be angled with respect to a stem of the medical device.

The portion can have a curled shape, a widened shape, a coiled shape, or a wave shape.

The portion can be coated with a hydrophilic coating.

The hydrophilic coating on a portion of the body can be coated with a hydrophobic coating.

The portion can be, or include, a weeping balloon, or a balloon that is inflatable and/or deflatable.

The weeping balloon can be configured to enlarge when filled with the medium.

The portion can include a biodegradable foam.

The portion can include a foam and/or a pledget that can be configured to enlarge upon absorption of the medium.

The portion can be rigid, or can include a rigid element.

The portion can be configured such that the medical device can anchor or pierce the Haller cells (ethmoid air cells).

The portion can be configured to be positioned in a frontal sinus, a maxillary sinus, or a sphenoid sinus.

A system includes a medical device and a delivery system comprising a connecting portion for removably connecting to a connecting portion of the medical device. The medical device includes a body, at least a first portion of the body being configured to be insertable within a target anatomical region, and at least a second portion of the body being configured to facilitate retention of the body in the target anatomical region. The delivery system facilitates transfer of a medium toward the medical device.

The system of the preceding paragraph can optionally include, additionally and/or alternatively, any one or more of the following features, configurations and/or additional components:

At least one of the first portion or the second portion of the medical device can be configured to interact with a portion surrounding the target anatomical region and/or configured to exchange a component to effect a change within the target anatomical region.

At least one of the first portion or the second portion of the medical device can be configured to receive a medium and permit exchange thereof with the target anatomical region, and wherein at least one of the first portion or the second portion of the medical device being configured to facilitate in situ manipulation of the medium via an external manipulation system.

The system can further include an external device positioned adjacent to the delivery system and/or the medical device.

The external device can be a force transducer.

The external device can be a magnet.

The system can further include an internal device positioned in the delivery system.

The internal device can be a magnetic wire that extends through the delivery system.

The medical device can further include a connecting portion for facilitating a removable connection between the body and the delivery system for delivering the medium.

The connecting portion on the delivery system can be a magnetic connecting portion, and wherein the connecting portion on the medical device can be a magnetic connecting portion.

The magnetic connecting portion on the delivery system can be configured to releasably couple to the magnetic connecting portion on the medical device.

The delivery system can further include a dispenser configured to receive the medium; and a delivery member with a proximal end attached to the dispenser; wherein the connecting portion of the delivery system can be connected to a distal end of the delivery member.

The delivery member can include flaps that are configured to open after a pressure in the delivery member exceeds a threshold pressure.

The system can further include a pressure sensor connected to the delivery member that can be configured to measure a pressure inside of the delivery member.

The delivery member and the dispenser can be integrally formed out of flexible material and the delivery member can be configured to expand.

The dispenser can be a syringe, a gas cylinder, a squeeze bottle, or a hand-held dispenser, with or without a trigger.

A system includes a medical device and a delivery device removably connectable to the medical device, the delivery device being configured to maintain the medical device in an insertion configuration during insertion of the medical device inside the target anatomical region. The medical device includes a body, at least a first portion of the body being configured to be insertable within a target anatomical region, and at least a second portion of the body being configured to facilitate retention of the body in the target anatomical region. The delivery device being further configured to decouple the medical device from the delivery system after insertion of the medical device in the target anatomical region.

The system of the preceding paragraph can optionally include, additionally and/or alternatively, any one or more of the following features, configurations and/or additional components:

At least one of the first portion or the second portion of the medical device can be configured to interact with a portion surrounding the target anatomical region and/or configured to exchange a component to effect a change within the target anatomical region.

At least one of the first portion or the second portion of the medical device can be configured to receive a medium and permit exchange thereof with the target anatomical region, and at least one of the first portion or the second portion of the medical device can be configured to facilitate in situ manipulation of the medium via an external manipulation system.

The medical device can further include a connecting portion for facilitating a removable connection between the body and a delivery system for delivering the medium.

A system includes a dispenser configured to receive a fluid, a delivery member with a proximal end attached to the dispenser and a distal end that includes a connecting member and a medical device that can be configured to be positioned in a body cavity. The medical device includes a body extending from a proximal end to a distal end, a connecting member connected to the body at the proximal end that can be configured to mate to the coupler of the delivery member, and a portion formed on the body adjacent to the distal end that can be configured to receive the fluid and deliver the fluid into a body cavity.

The system of the preceding paragraph can optionally include, additionally and/or alternatively, any one or more of the following features, configurations and/or additional components:

The connecting member on the delivery member can include a magnetic connecting member, and the connecting member on the medical device can include a magnetic connecting member.

The magnetic connecting member on the delivery member can be configured to releasably couple to the magnetic connecting member on the medical device.

The delivery member can include flaps that are configured to open after a pressure in the delivery member exceeds a threshold pressure.

The system can further include a pressure sensor connected to the delivery member that can be configured to measure a pressure inside of the delivery member.

The delivery member and the dispenser can be integrally formed out of flexible material and the delivery member can be configured to expand.

The dispenser can include a syringe, a gas cylinder, a squeeze bottle, or a hand-held dispenser, with or without a trigger.

The system can further include an external device positioned adjacent to the delivery member and/or the medical device.

The external device can be a force transducer.

The external device can be a magnet.

The system can further include an internal device positioned in the delivery member.

The internal device can be a magnetic wire that extends through the delivery member.

A system includes a medical device that can be configured to be positioned in a body cavity to deliver a medium to the body cavity, and an external device positioned adjacent to the medical device.

The system of the preceding paragraph can optionally include, additionally and/or alternatively, any one or more of the following features, configurations and/or additional components:

The medical device can be a fluid that undergoes a transformation to a scaffold upon application of a force.

The external device can be a force transducer.

A method of inserting a medical device into a sinus cavity includes deploying a distal end of a delivery device into a nasal cavity, positioning the distal end of the delivery device near a sinus ostium, advancing a guide wire of the delivery device through the sinus ostium and into the sinus cavity, advancing a medical device over the guide wire into the sinus cavity, wherein the medical device includes a body with a connecting portion at a proximal end and a portion at a distal end, and removing the guide wire from the sinus cavity, wherein the medical device remains in place in the sinus cavity.

The method of the preceding paragraph can optionally include, additionally and/or alternatively, any one or more of the following features, configurations and/or additional components:

The method can further include visualizing the sinus cavity; and cleansing the sinus cavity.

The method can further include removing the delivery device from the nasal cavity.

The method can further include coupling a dispenser to the medical device; delivering a fluid to the medical device; delivering the fluid to the sinus cavity through the medical device; and decoupling the dispenser from the medical device.

The connecting portion on the medical device can be a magnetic connecting portion, and wherein coupling a dispenser to the medical device includes coupling a magnetic connecting portion on the dispenser to the magnetic connecting portion on the medical device.

The fluid can be a therapeutic fluid.

The fluid can be a cleansing agent.

Delivering the fluid to the sinus cavity through the medical device can include delivering the fluid to the sinus cavity through pores on the medical device.

Delivering the fluid to the sinus cavity through the medical device can include eluting the fluid from the medical device into the sinus cavity.

The method can further include applying an external field to the fluid.

Applying an external field to the fluid can include applying a magnetic field to the fluid.

Applying an external field to the fluid can include applying a force field to the fluid.

The method can further include applying a light source, or light emitted by a light source, to the fluid.

A method includes coupling a dispenser to a medical device positioned in a cavity, delivering a fluid to the medical device, wherein the medical device includes a body with a connecting portion at a proximal end and a portion at a distal end, delivering the fluid to the cavity through the medical device, and decoupling the dispenser from the medical device.

The method of the preceding paragraph can optionally include, additionally and/or alternatively, any one or more of the following features, configurations and/or additional components:

The connecting portion on the medical device can be a magnetic connecting portion, and wherein coupling a dispenser to the medical device includes coupling a magnetic connecting portion on the dispenser to the magnetic connecting portion on the medical device.

The fluid can be a therapeutic fluid.

The fluid can be a cleansing agent.

Delivering the fluid to the cavity through the medical device can include delivering the fluid to the cavity through pores on the medical device.

Delivering the fluid to the cavity through the medical device can include eluting the fluid from the medical device into the cavity.

The method can further include applying an external field to the fluid.

Applying an external field to the fluid can include applying a magnetic field to the fluid.

Applying an external field to the fluid can include applying a force field to the fluid.

The method can further include applying a light source, or light emitted by a light source, to the fluid.

## Claims

1. An at least partially implantable medical device, the medical device comprising:
a body;
at least a first portion of the body configured to be implanted through an ostial opening; and
at least a second portion of the body configured to at least temporarily retain the body in the implanted position, the second portion more distal than the first portion and including a cross-sectional area larger than a cross-sectional area of the first portion; and
at least one of the first portion or the second portion configured to elute a fluid.

2. The medical device of claim 1, wherein the second portion is angled relative to the first portion.

3. The medical device of any of claims 1 to 2, wherein the second portion has a widened shape or a wave shape.

4. The medical device of any of claims 1 to 3, wherein at least a portion of the body is coated with a hydrophilic material.

5. The medical device of claim 4, wherein the hydrophilic coating on the body is coated with a hydrophobic coating.

6. The medical device of any of claims 1 to 5, wherein the second portion includes an inflatable and deflatable balloon.

7. The medical device of any of claims 1 to 6, wherein the second portion includes a foam and/or a pledget configured to enlarge upon absorption of a medium.

8. The medical device of any of claims 1 to 7, wherein the second portion includes a rigid member.

9. The medical device of claim 8, wherein the second portion is configured to anchor or pierce tissue.

10. A system comprising:
an at least partially implanted medical device including:
a body;
at least a first portion of the body configured to be implanted; and
at least a second portion of the body configured to retain the body in the implanted position; and
a delivery system comprising a connecting portion for removably connecting to a connecting portion of the medical device;
wherein the delivery system facilitates transfer of a medium toward the medical device, wherein at least one of the first portion or the second portion of the medical device is configured to receive a medium and permit eluting of the medium in the implanted position, and wherein at least one of the first portion or the second portion of the medical device is configured to facilitate *in situ* manipulation of the medium via an external manipulation system.

11. The system of claim 10, further comprising an external device positioned adjacent to the delivery system and/or the medical device.

12. The system of claim 11, wherein the external device is a force transducer and/or a magnet.

13. The system of any of claims 10 to 12, further comprising an internal device positioned in the delivery system.

14. The system of claim 13, wherein the internal device is a magnetic wire that extends through the delivery system.

15. The system of any of claims 10 to 14, wherein the in situ manipulation of the medium includes magnetizing the medium to align magnetic particles of the medium.
